# EUROPEAN PATENT SPECIFICATION

(11) **EP 4 188 912 B1**
(45) Date of publication and mention of the grant of the patent: **03.07.2024**
(21) Application number: 21752511.2
(22) Date of filing: 26.07.2021
(51) Int. Cl.: C07D 257/02

(54) **MANUFACTURING OF DIMERIC CONTRAST AGENTS**
HERSTELLUNG VON DIMEREN KONTRASTMITTELN
FABRICATION D'AGENTS DE CONTRASTE DIMÉRIQUES

(30) Priority: 27.07.2020 EP 20187948
(43) Date of publication of application: 07.06.2023
(73) Proprietor: Bracco Imaging SpA, 20134 Milano (IT)
(72) Inventor: BARALE, Andrea, 10010 Colleretto Giacosa (IT); BOI, Valeria, 10010 Colleretto Giacosa (IT); FERRIGATO, Aurelia, 10010 Colleretto Giacosa (IT); GAZZETTO, Sonia, 10010 Colleretto Giacosa (IT); GIARDINA-PAPA, Daniela, 33050 Torviscosa (IT); MORTILLARO, Armando, 10010 Colleretto Giacosa (IT); ORIO, Laura, 10010 Colleretto Giacosa (IT); VERANI, Massimo, 10010 Colleretto Giacosa (IT)
(74) Representative: Ravizza, Claudio
(86) International application number: PCT/EP2021/070801
(87) International publication number: WO 2022/023240

(56) References cited:
- WO-A1-2017/098044

## Description

### FIELD OF THE INVENTION

The present invention relates to the preparation of contrast agents for use in Magnetic Resonance Imaging (MRI). In particular the invention relates to a new process for the preparation of dimeric contrast agents, especially [µ-[1-[bis[2-(hydroxy-κ*O*)-3-[4,7,10-tris[(carboxy-κ*O*)methyl]-1,4,7,10-tetraazacyclododec-1-yl-κ*N*¹,κ*N*⁴,κ*N*⁷,κ*N*¹⁰]propyl]amino]-1-deoxy-D-glucitolato(6-)]]di-Gadolinium complex.

### STATE OF THE ART

Magnetic Resonance Imaging (MRI) is a renowned diagnostic imaging technique increasingly used in clinical diagnostics for a growing number of indications.

The strong expansion of medical MRI has further benefited from the development of a class of compounds, the MRI contrast agents, that act by causing a dramatic variation of nearby water proton relaxation rates in the tissues/organs/fluids wherein they distribute, thus adding relevant physiological information to the impressive anatomical resolution commonly obtained in the uncontrasted MRI images.

Contrast agents for use in the MRI imaging technique typically include a paramagnetic metal ion which is complexed with a cyclic or acyclic chelating ligand, more typically a polyaminopolycarboxylic chelator. The most important class of MRI contrast agents is represented by the Gd(III) chelates which are currently used in about 1/3 of the clinical tests. Indeed, Gd(III) is highly paramagnetic with seven unpaired electrons and a long electronic relaxation time, making it an excellent candidate as a relaxation agent.

WO2017098044 (same applicant as the present application) discloses dimeric paramagnetic complexes useful as contrast agents, specifically in Magnetic Resonance Imaging (MRI), of formula 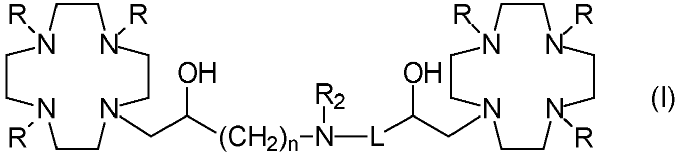 and a synthetic route for their preparation.

Among a number of specific compounds, the application discloses the di-gadolinium complex of the 1-[bis[2-hydroxy-3-[4,7,10-tris(carboxymethyl)-1,4,7,10-tetraazacyclododec-1-yl]propyl]amino]-1-deoxy-D-glucitol ligand of formula 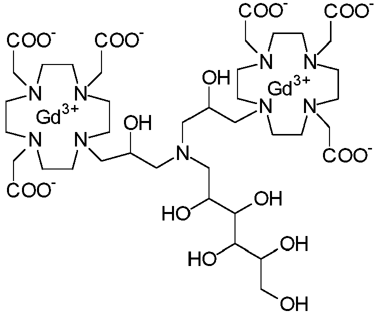 hereinafter otherwise identified as "dimeric complex compound **5"** or, more simply as "Compound 5".

This complex compound shows interesting properties, especially in terms of relaxivity and tolerability that makes it suitable for use in the *in vivo* diagnostic imaging carried out with doses of the paramagnetic complex lower than those required by the contrast agents of the market.

A preparation process is moreover disclosed in the above international application, schematized in the following general **Scheme 1:**

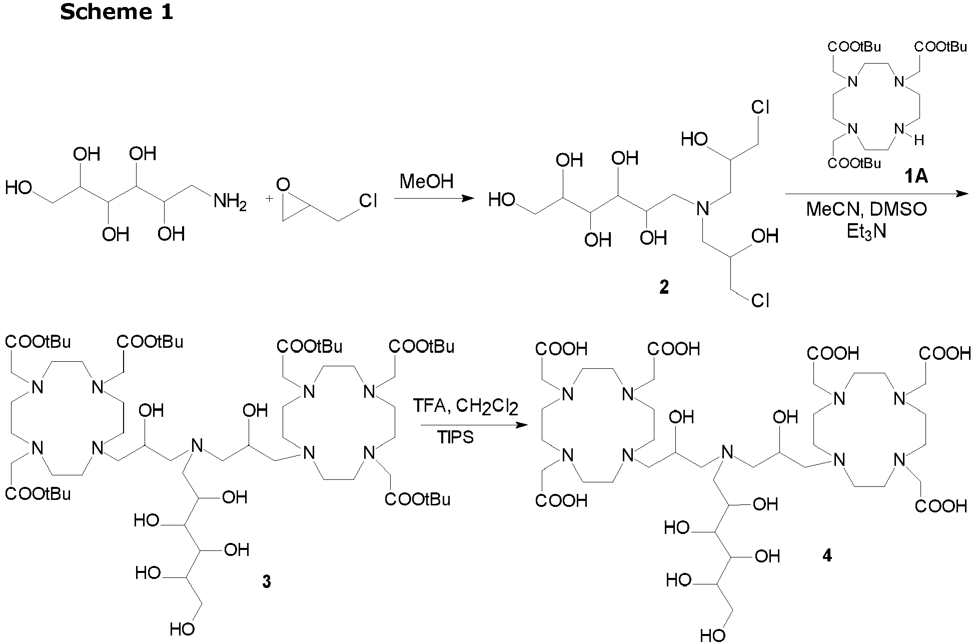

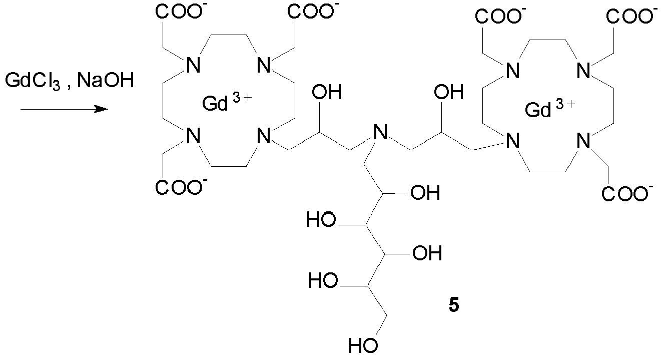

Main steps of the disclosed synthetic process comprise:
a) preparation and isolation of DO3A tri-tert-butyl ester (compound **1A)** carried out substantially as disclosed in Org. Synth. 2008, 85, 10;
b) preparation of the intermediate **2** by alkylation of D-glucamine with epichlorohydrin (molar ratio 1 : 4.95) in MeOH at 50 °C for 26 h, and the isolation of the condensation product by evaporation of the crude reaction;
c) alkylation of DO3A tri-tert-butyl ester with the intermediate **2** in DMSO and Et₃N, evaporation and purification of the crude residue on Amberlite XAD^{®}1600 leading to give the protected ligand **3;**
d) deprotection of the ligand **3** with TFA acid and TIPS in dichloromethane, evaporation of the crude reaction and purification of the residue on Amberlite XE 750;
e) complexation of the ligand **4** in water with gadolinium chloride hexahydrate, and purification on Amberchrome CG161M resin of the crude product obtained by filtration and evaporation of the solution.

This process requires the synthesis and isolation of each of the individual intermediates, which is generally carried out by evaporation to residue of the solvent. Such isolation steps, besides being unsuitable for a large-scale production, unavoidably result in a reduction in the overall yield and efficiency of the process. Moreover, the prior art process is not particularly suitable for working on larger scales, for example on industrial processes, because it encompasses the use of harsh materials that are difficult to handle, such TFA, TIPS and DCM, which might i.a. cause corrosion and thus wear out the synthesis apparatuses and/or might not be safe for the health of the workers.

### SUMMARY OF THE INVENTION

The present invention generally relates to an optimized process for the manufacturing of the dimeric complex compound **5** which includes preparation steps carried out one-pot and without isolation of the resulting intermediates which allow for both time saving and an improved overall yield and efficiency.

More particularly, the invention relates to a process for the manufacturing of the dimeric complex compound **5** 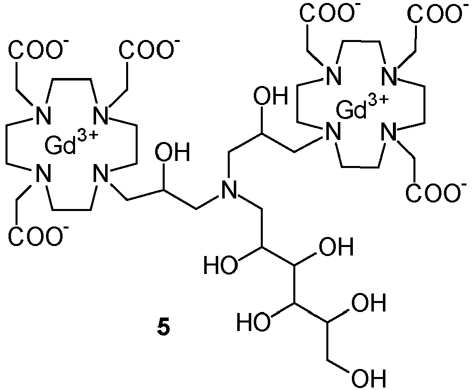 which comprises, as main steps:
**1)** preparing a solution of DO3A tri-tert-butyl ester of formula **1A** 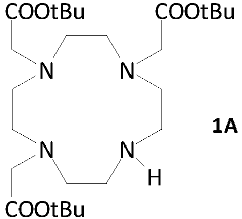 for example in an organic solvent;
**2)** preparing a solution of the intermediate of formula **2** 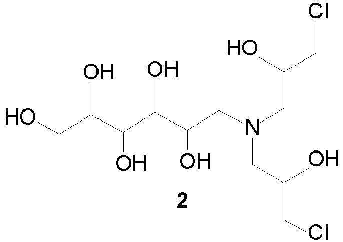 for example in an organic solvent;
**3)** admixing the solutions prepared according to steps **1)** and **2)** to obtain a solution of the protected ligand of formula **3** 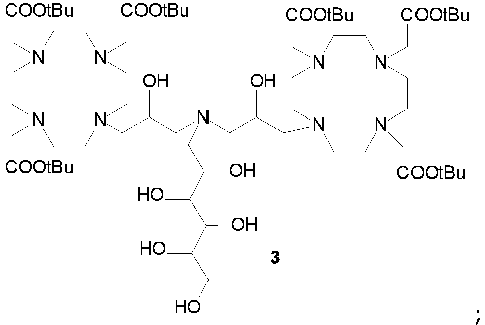
**4)** without isolating the protected ligand from the solution of step **3)** removing the tert-butyl protecting groups from the protected ligand to obtain a solution of a respective free ligand of formula **4** 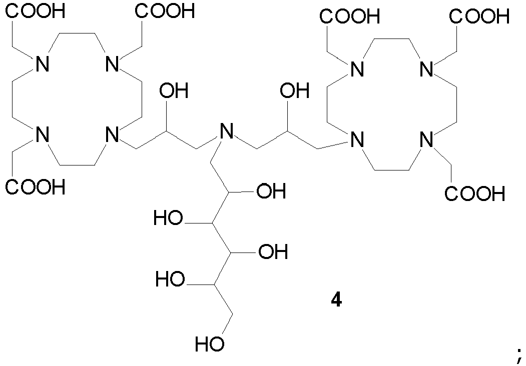
**5)** without isolating the free ligand of formula **4** from the obtained solution, adding gadolinium metal ions to the solution of step **4)** to obtain a solution of the respective dimeric complex of formula **5**; and
**6)** isolating the dimeric complex.

In a preferred aspect of the invention, the reaction solvent in all the steps following the preparation of the compound of formula **3** is an aqueous solvent or an aqueous solvent mixture. The aqueous solvent or aqueous solvent mixture advantageously does not comprise harsh materials such as TFA, TIPS and/or DCM.

The step **1)** of the process generally comprises the preparation of a solution of DO3A tri-tert-butyl ester **1A.** In one embodiment the solution is prepared in an organic solvent, e.g. by solubilizing in the solvent a commercial DO3A tri-tert-butyl ester, or a DO3A tri-tert-butyl ester prepared by using a known synthetic procedure.

In a preferred embodiment the solution of **1A** is prepared just before its use, by converting a salt of the DO3A tri-tert-butyl ester, such as a hydrobromide salt to the respective free base **1A.** The conversion, typically comprising the neutralization of the hydrobromide, is preferably carried out in an organic solvent and in the presence of a base or a basic salt (a salt that is the product of the neutralization of a strong base and a weak acid, which hydrolyzes by forming a basic solution). Then, the removal of formed salts and optional concentration of the filtered solution allows to achieve a solution of the DO3A tri-tert-butyl ester **1A** in the organic solvent that is suitable for use as such in the subsequent step of the process, without requiring any purification or isolation of the ester.

Suitable organic solvents preferably include MeCN, propylene carbonate, ethanol, t-butanol, hexane, and the like. More preferably, the organic solvent is MeCN.

Suitable bases or basic salts for the neutralization of the starting hydrobromide for instance include strong bases and anion exchange resins such as, for example, Diaion PA308, Amberlite IRA 400, KOH, tBuOK, Na₂CO₃ and K₂CO₃, where the latter two are preferred. More preferably, the neutralization of the DO3A tri-tert-butyl ester hydrobromide is carried out in the presence of K₂CO₃.

The step **2)** of the process comprises the preparation of the compound of formula **2,** which may be obtained by alkylation of D-glucamine with epichlorohydrin. The alkylation is carried out in organic solvent, such as a dipolar organic solvent or in an aqueous mixture thereof. Suitable organic solvents for instance include DMAC, DMF, alcohols such as MeOH, and their mixtures. More preferably, the organic solvent is DMAC. The distillation of any aqueous solvent and/or epichlorohydrin excess from the mixture leads then to achieve a solution of the compound of formula **2** in the organic solvent which is suitable for use as such in the next step of the process, without isolation and/or further purification of the alkylation product.

The step **3)** of the process essentially comprises the condensation (or coupling, as herein used interchangeably) of the intermediate compound of formula **2** with the DO3A tri-tert-butyl ester **1A** with formation of the protected ligand of formula **3.** The condensation reaction is preferably carried out in the presence of a base, e.g. acting as an acceptor of the formed HCl. Suitable bases for instance include anion exchange resins such as Amberlite GC 400, NMM, tBuOK, Et₃N, and DIPEA, wherein Et₃N and DIPEA are preferred and DIPEA is particularly preferred.

In one embodiment the condensation reaction is carried out by addition of the base and the organic solution of DO3A tri-tert-butyl ester **1A** directly collected from step **1)** to the solution of the compound **2** collected from step **2),** to give an organic crude solution comprising the condensation product of formula **3** in an organic solvent mixture. Then, a purification of the organic crude leading to obtain the purified product in a water/organic solvent mixture, and the optional final distillation of any organic solvent allow to achieve the protected ligand of formula **3** in an aqueous solvent or an aqueous solvent mixture which can be used as such in the next step of the process, without isolation and/or further purification of the protected ligand itself.

The step **4)** of the process substantially comprises the removal of the carboxyl protecting groups from the protected ligand of formula **3** to give an aqueous solution or an aqueous mixture of the respective free ligand of formula **4.** The deprotection by hydrolysis of tert-butyl protecting groups can be carried out in both acidic and basic conditions, by using reactants and conditions known to those skilled in the relevant art. In one embodiment, the deprotection is carried out by acidification of the aqueous solution or aqueous mixture of the protected ligand directly collected from step **3)** of the process, to achieve an acidic solution of the free ligand of formula **4.** The acidification is preferably carried out by addition of an acid, for instance selected from HCl, H₂SO₄, and H₃PO₄. In a preferred embodiment the deprotection is performed by using HCl. Then, the neutralization of the acidic solution, subsequent purification and partial concentration of the resulting mixture lead to collect an aqueous solution or aqueous mixture of the ligand **4,** that is used as such in the complexation step, without isolation.

The step **5)** comprises the complexation of the ligand with gadolinium metal ions, to the desired dimeric complex **5.** The complexation reaction can conveniently be carried out according to know procedures, for instance by stoichiometric addition of a suitable Gd(III) derivative, particularly an oxide such as Gd₂O₃ or of a gadolinium salt to the solution of the ligand. In one embodiment the complexation reaction is carried out by addition of GdCl₃ to the solution of the ligand directly collected from step **4)** of the process. The resulting mixture is adjusted to a pH value of from about 5 to about 7 and maintained under stirring to give an aqueous solution or aqueous mixture of the gadolinium complex **5** that is then purified and concentrated to achieve solution of the desired dimeric complex **5** having the desired purity.

The step **6)** comprises the final isolation of desired gadolinium complex **5.** This step can conveniently be carried out according to know procedures. In one embodiment the solution of the purified complex collected from step **5)** is spray-dried to give the desired product as a white solid satisfying the required purity specifications.

Interestingly the above process avoids or strongly reduces the use of harsh reagents, such as trifluoroacetic acid (TFA), and nasty solvents, such as dichloromethane, which are required in the process of the prior art and are difficult to handle when working on larger scales, for example on industrial processes.

Moreover, it comprises steps that are carried out one-pot, which are suitable for a large-scale implementation, and which do not require the isolation of any of the prepared precursor (such as **1A)** or intermediates. As a result, in addition to promote a reduction in process times, and of being easily implemented on larger scales as stated above, the process advantageously allows a significant increase in the overall process yield, from 10% (obtained with the process disclosed in WO2017098044) up to an overall yield of at least 20%, preferably of 25%, typically of about 28%, advantageously over 30%.

### DETAILED DESCRIPTION OF THE INVENTION

In the present description, and unless otherwise provided, the term "intermediate" (e.g. used with reference to the compound of formula **2** deriving from the alkylation reaction of the D-glucamine with epichlorohydrin, or the protected ligand of formula **3)** comprises within its meaning a molecule produced in the course of a chemical synthesis or preparation step of the process which is not itself the final product, but requires one (or more) further reactions e.g. alkylation/deprotection/complexation reaction(s) to give the final product of the process, namely the dimeric complex compound **5.**

Unless otherwise provided, the term "precursor" (e.g. used with reference to the compound **1A**) comprises within the meaning a molecule that participates in a chemical reaction promoting its transformation into another molecule, which includes or is derived from said precursor.

In the present description, the term "aqueous solvent" comprises within the meaning water and aqueous saline solutions, possibly including small amounts of organic solvents miscible with water, such as a volume percentage of 10% or lower of organic solvents miscible with water, preferably 8% or lower, and more preferably 5% or lower, for example because the process of the invention is carried out without isolating most of the intermediate products, thereby small amounts of organic solvents can be carried on through the upstream and first steps of the process. Preferably the aqueous solvent is water.

The expression "water/organic solvent mixture" or, more simply, "aqueous solvent mixture" as used herein interchangeably, comprises within the meaning a mixture of two or more solvents which comprises an aqueous solvent, such as a mixture of water and one or more organic solvents all miscible with each other, to give a homogeneous solvent mixture, wherein the volume percentage of the one or more organic solvents is higher than 10%, preferably higher than 15%, more preferably higher than 20%. Suitable examples include mixtures of water and acetonitrile (or water/MeCN) used as eluents in the chromatographic purifications e.g. of the compound of formula **3,** or the mixture of water/MeCN /DMAC e.g. resulting after dilution with water of the crude mixture resulting from the condensation reaction of step **3).** According to a preferred aspect of the present invention, the one or more organic solvents within the aqueous solvent mixture (as well as within the aqueous solvent, when present) is not a harsh solvent or material; indeed, the aqueous solvent mixture preferably does not comprise harsh solvents or materials such as TFA, TIPS and/or DCM.

Likewise, the expressions "aqueous solution" and "aqueous mixture" include in their meaning, respectively, a solution or a mixture containing water. Suitable examples include, respectively, a solution of one or more compounds, e.g. a reagent, an acid, a base or a reaction product in water (more in general, in an aqueous mixture) or in an aqueous solvent mixture, and a mixture, such as the water/organic mixture resulting from the addition of water or an aqueous solution to a reaction mixture in an organic solvent or solvent mixture.

In the present description the term "protecting group" (e.g. used with reference to the compound of formula **3)** designates a protective group adapted for preserving the function of the group to which it is bound. Specifically, protective groups are used to preserve carboxyl functions. More specifically the term designates tert-butyl groups preserving the chelating function of carboxyl groups of the ligand by formation of tert-butyl esters [see, for a general reference on protecting groups and deprotecting conditions, T. W. Green and P. G. M. Wuts; Protective Groups in Organic Synthesis, Wiley, N.Y. 1999, third edition].

An embodiment of the invention relates to a process for the manufacturing of the dimeric compound **5** essentially as schematized in the following general synthetic **Scheme 2** 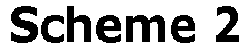 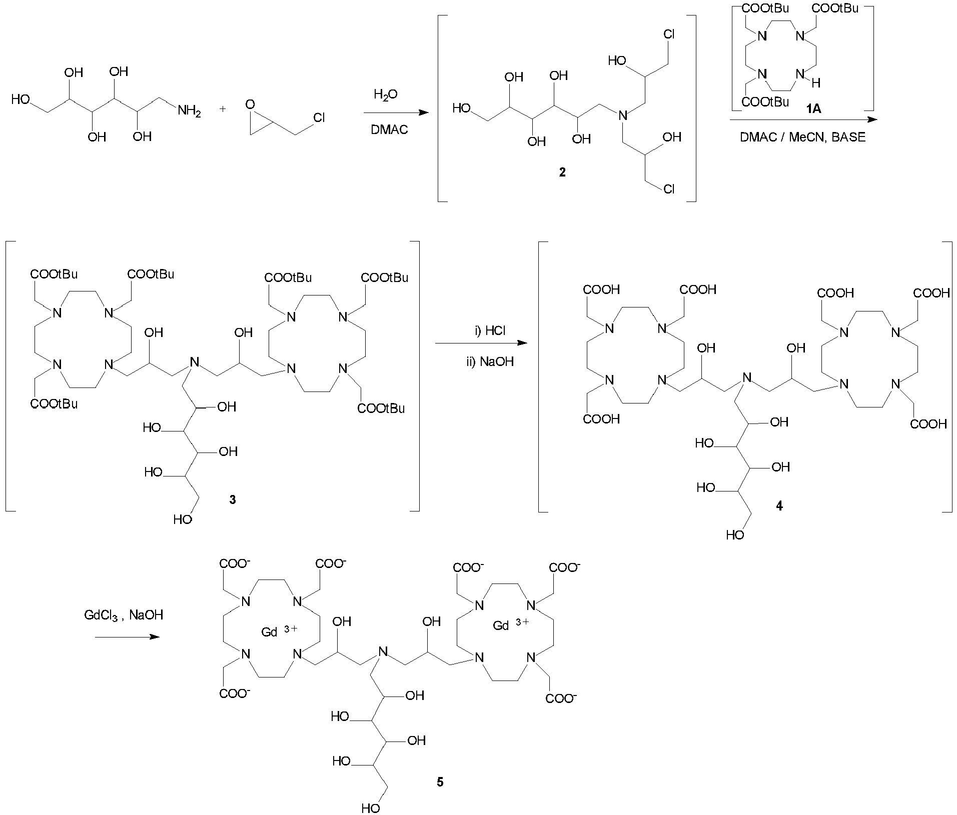 which comprises, as main steps:
1) neutralizing a hydrobromide salt of the DO3A tri-tert-butyl ester in an organic solvent, to give a solution of DO3A tri-tert-butyl ester **1A** in the organic solvent;
2) reacting D-glucamine with epichlorohydrin to obtain a solution of the compound of formula **2** in an organic solvent such as DMAC; and, without isolation of the product
3) reacting the compound of formula **2** from step **2)** with DO3A tri-tert-butyl ester **1A** from step **1)** in the presence of a base and optional concentration of the solution to give an organic crude, dilution of the organic crude with water, a water/organic solvent mixture and/or an optional aqueous solution to obtain a water/organic crude, purification of the water/organic crude and optional removal of any organic solvent to give an aqueous solution or an aqueous mixture of the protected ligand of formula **3;** and, without isolation of the product
4) acidifying the solution of the protected ligand of formula **3** from step **3)** to give an acidic aqueous solution or aqueous mixture of the respective deprotected ligand **4,** neutralizing the acidic solution and purifying the resulting neutral solution to give an aqueous solution or aqueous mixture of the deprotected ligand **4,** and, without isolation of the latter,
5) adding gadolinium metal ions to the solution of the ligand **4** to obtain a solution of the corresponding complex compound **5**; and
6) isolating the complex.

### Step 1

The first step of the process comprises preparing a solution of the the DO3A tri-tert-butyl ester **1A** in an organic solvent, such as MeCN, by converting a hydrobromide salt of the DO3A tri-tert-butyl ester to the respective free base directly in the organic solvent, in the presence of a base or a basic salt.

In a preferred embodiment the step **1)** of the process comprises:
i) suspending a hydrobromide salt of the DO3A tri-tert-butyl ester together with a base or a basic salt such as K₂CO₃ in an organic solvent such as MeCN to obtain a suspension comprising formed salts;
ii) filtering the suspension; and
iii) collecting and optionally concentrating the filtered suspension, to obtain a solution of the DO3A tri-tert-butyl ester **1A** in the organic solvent which is suitable for use as such in the subsequent condensation reaction, without requiring any purification or isolation of ester.

The ester hydrobromide and K₂CO₃ are preferably suspended in the organic solvent at room temperature to give a mixture that is then maintained under stirring at a temperature of from 20 to 30 °C, preferably of about 25 °C for a time of from 16 to 30 h, preferably for 18-20 h. The mixture is then treated to remove the formed salts, preferably by filtration.

In a preferred embodiment, the solution resulting by filtration is subjected to a thermal concentration, for example by partial distillation of the solvent, to give a solution of the tri-tert-butyl ester **1A** in MeCN with a final concentration of from 55 to 65% and preferably of about 60% (w/w) which is suitable for use as such in the subsequent condensation step of the process.

### Step 2

The step comprises preparing a solution of the intermediate compound of formula **2** by reacting D-glucamine with epichlorohydrin. In one embodiment the reaction is carried out in a mixture of solvents, preferably in water/DMAC, by using a slight excess of epichlorohydrin over the stoichiometric amount, for example of from 2 to 3 and more preferably of about 2.2 moles of epichlorohydrin per mole of D-glucamine. Preferably, such reaction of step **2)** is carried by using a slight excess of epichlorohydrin over the stoichiometric amount, for example from 2 to 3 moles, more preferably from 2.05 to 2.5 moles, and even more preferably about 2.2 moles of epichlorohydrin per mole of D-glucamine.

In a preferred embodiment the step **2)** of the process comprises:
i) adding an aqueous solution of D-glucamine to a solution of epichlorohydrin in DMAC to give the intermediate compound of formula **2** in a water/DMAC solvent mixture; and
ii) removing the water from the solvent mixture, to obtain a solution of the compound of formula **2** in the organic solvent.

The addition of D-glucamine to the solution of epichlorohydrin is preferably carried out at room temperature, in a time of about 2 h, to give a mixture that is kept under stirring at a temperature of from 15 to 30 °C, preferably from 15 to 25 °C and more preferably from 20 to 25 °C, for a time of from 16 to 24 h, preferably from 16 to 20 h and more preferably of about 17 h.

The mixture is then distilled, to remove water and any optional epichlorohydrin residue. The distillation is preferably carried out at reduced pressure, and a temperature preferably of 40-65 °C, leading to achieve a solution of the desired intermediate compound of formula **2** in DMAC with a residual water content preferably < 2% w/w. The achieved solution is then used as such in the subsequent condensation reaction, without requiring any isolation or purification of the product.

### Step 3

The step essentially comprises the condensation of the DO3A tri-tert-butyl ester **1A** with the intermediate **2,** in the presence of a base such as Et₃N or, more preferably, DIPEA. The condensation is preferably carried out by admixing the base and the solutions of the ester **1A** in MeCN from step **1)** with the solution of the intermediate **2** in DMAC directly collected from step **2)** to give a raw solution (or crude) comprising the protected ligand of formula **3** in the mixture of the MeCN/DMAC organic solvents that is then purified.

In one embodiment, the organic crude solution resulting from the condensation reaction is diluted with water or is partially concentrated and then diluted with water or an aqueous solvent mixture, preferably water/MeCN, to obtain a water/organic crude, or aqueous crude, as herein used interchangeably.

In a preferred embodiment, the water/organic crude thus obtained or, preferably, the organic crude resulting from the condensation reaction are added with an aqueous solution which promotes the precipitation of reaction salts, including DO3A tri-tert-butyl ester hydrochloride, which are then removed by filtration, to provide a water/organic filtered solution. The water/organic crude or the water/organic filtered solution are then purified, preferably by chromatography.

In one embodiment, the aqueous solution used to promote the precipitation of the hydrochloride salts is aqueous ammonia.

More particularly, the step **3)** of the process preferably comprises:
i) condensation of the intermediate compound of formula **2** from step **2)** with DO3A tri-tert-butyl ester **1A** from step **1)** in the presence of a base, preferably DIPEA, to give an organic crude solution comprising the condensation product of formula **3** and reaction salts in the organic solvent mixture, and optional concentration of the organic crude;
ii) dilution of the organic crude of step i) with water or a water/organic solvent mixture, preferably water/MeCN, to give a water/organic crude;
iii) optional addition to the water/organic crude of an aqueous solution promoting the precipitation of reaction salts that are removed by filtration to give a water/organic filtered solution; or
iv) dilution of the organic crude of step i) with the aqueous solution promoting the precipitation of reaction salts that are removed by filtration, to give a water/organic filtered solution;
v) purification of the water/organic crude of step ii), or of the water/organic filtered solution of steps iii) or iv) to give a solution of the protected ligand of formula **3** in a water/organic solvent mixture that can be used in the next deprotection step of the process without requiring any isolation or further purification of the protected product; and
vi) optional removal of any organic solvent from the mixture, to obtain a solution of the protected ligand of formula **3** in water that is used in the next deprotection step of the process without requiring any isolation or further purification of the protected product.

The condensation reaction is preferably carried out by addition of the base and a solution of the ester **1A** in MeCN collected from step **1)** of the process to the solution of the intermediate compound of formula **2** in DMAC collected from step **2).**

Suitable amounts of base and ester **1A** are conveniently determined with respect to the amount of D-glucamine subjected to the reaction. In one embodiment, the condensation reaction is carried out by using from 1.6 to 2.4 moles and preferably about 1.8 moles of ester **1A,** and from 2 to 4 moles, preferably about 2.3 moles of DIPEA per mole of starting D-glucamine subjected to reaction.

The addition is preferably performed at a temperature of 40-50 °C. The condensation reaction is then carried out at a temperature of from 50 to 80 °C, preferably from 65 to 75 °C for a time e.g. of 60-80 h, preferably of 70-75 h, to give a raw solution comprising the desired condensation product of formula **3** and hydrochloride salts in a MeCN/DMAC solvent mixture.

In one embodiment, the raw solution is then diluted with water to give a water/organic crude having, preferably, a concentration of about 25-30%, more preferably of about 25% (w/w). In one preferred embodiment, the aqueous crude comprises an amount of water which by weight is at least equal to the amount of the organic solvent, specifically MeCN in the mixture; more preferably the crude has a water:MeCN ratio of about 60:40.

The water/organic crude is then purified, preferably by chromatography, more preferably on resins, even more preferably on adsorbent resins, such as Amberlite XAD^{®} 1600. In a preferred embodiment, the aqueous crude is purified on an adsorbent resin, such as Amberlite XAD^{®} 1600, by using a water/MeCN mixture as eluent, allowing to achieve both the unreacted DO3A tri-tert-butyl ester **1A** and the pure condensation product as separated fractions in a water/MeCN solvent mixture.

In another embodiment the raw solution resulting from the condensation reaction is first concentrated by removing at least a part of the MeCN, e.g. by distillation. The concentrated solution is then diluted with water or with a mixture of water: MeCN allowing to obtain a water/organic crude having the above water: MeCN ratio, that is then purified by chromatography, as above said.

Optionally, the water/organic crude obtained as above said is added with an aqueous solution such as aqueous ammonia which promotes, by cooling of the mixture, the precipitation of the unreacted DO3A tri-tert-butyl ester as hydrochloride, that is then removed by filtration and, optionally recycled. The filtered solution devoid of most of the chloride salts is then purified by chromatography on an adsorbent resin, such as Amberlite XAD^{®} 1600 resin as above said, to give the residual DO3A tri-tert-butyl ester **1A** and the pure condensation product as separated fractions in a water/organic, such as a water/MeCN solvent mixture.

In a preferred embodiment, the aqueous solution, e.g. including aqueous ammonia is added directly into the organic raw solution resulting from the condensation reaction promoting, by cooling of the mixture, the precipitation of the unreacted DO3A tri-tert-butyl ester as hydrochloride that is then removed by filtration and, optionally recycled. The filtrate devoid of most of the chloride salts is then purified by chromatography on an adsorbent resin, such as Amberlite XAD^{®} 1600 resin as above said, to give the residual DO3A tri-tert-butyl ester **1A** and the pure condensation product as separated fractions in a water/organic, such as a water/MeCN, solvent mixture.

The optional final distillation of the organic solvent, e.g. under reduced pressure, from pure fractions leads then to achieve the condensation product of formula **3** in an aqueous solution with a final concentration of 5-15% (w/w), preferably of about 10% (w/w), that is suitable for use in the subsequent deprotection step as such, without requiring any isolation or additional purification of the intermediate.

Interestingly, the above procedural steps allow to obtain the protected condensation product **3** in an aqueous solvent or aqueous solvent mixtures, thus making it possible to carry out its deprotection and complexation to the final complex **5** by using water, and more in general aqueous solvents or aqueous solvent mixtures, as the only or one of the main reaction solvents. The protected condensation product **3** in an aqueous solvent can be obtained according to various methods known to the skilled person. For example, as stated above, the organic crude solution of compound of formula **3,** e.g. obtained by reacting the compound of formula **2** from step **2)** with DO3A tri-tert-butyl ester **1A** from step **1),** can be diluted with water, a water/organic solvent mixture, or an aqueous solution, thus obtaining a water/organic crude. Before removal of the organic solvent, the water/organic crude can be purified via chromatography, preferably via a resin, and more preferably via an adsorbent resin, such as Amberlite XAD^{®} 1600. Then, the organic solvent can be removed to obtain the aqueous solution of the compound of formula **3,** for example by distillation e.g. under reduced pressure. Also the protected condensation product **3** in an aqueous solvent mixture can be achieved according to various methods known to the skilled person. For example, the organic crude solution of compound of formula **3,** e.g. obtained by reacting the compound of formula **2** from step **2)** with DO3A tri-tert-butyl ester **1A** from step **1),** can be diluted with water, a water/organic solvent mixture, or an aqueous solution, thus obtaining a water/organic solution to be used in the subsequent steps without isolation of the product **3.**

Therefore, according to a preferred aspect of the present invention, the present invention comprises the further step of converting the solution of a compound of formula **3** to an aqueous solution of, or to an aqueous mixture of, a compound of formula **3.** Preferably, this further step is carried out by: (i) diluting the solution of a compound of formula **3** with water, a water/organic solvent mixture, or an aqueous solution, thus obtaining an aqueous mixture (or a water/organic solution), and (ii) optionally removing the organic solvent, e.g. by distillation, thus obtaining an aqueous solution.

### Step 4

This step comprises the deprotection of the protected ligand of formula 3 by removing carboxyl protecting groups leading to achieve an aqueous solution or aqueous mixture of the respective free ligand **4.** The reaction is preferably carried out by acidification of the aqueous solution or mixture of protected ligand of formula **3** directly collected from step **3)** of the process.

In one embodiment the step **4)** of the process comprises:
i) Addition of an acid to the aqueous solution or aqueous mixture of the compound of formula **3** collected from step **3)** to achieve acidic solution of the free ligand **4;**
ii) Addition of a base to the acidic solution, to achieve a substantially neutralized solution of the ligand **4;**
iii) Purification of the neutralized solution and subsequent optional concentration, to give an aqueous solution or aqueous mixture of the free ligand **4** that is suitable for use as such in the next complexation reaction, without requiring any isolation of the ligand.

In one embodiment, the solution of the protected compound of formula **3** is acidified by addition of an acid, such as 34% aqueous HCl. The acidification is performed by using a large excess of HCl, e.g. from 30 to 100, preferably from 30 to 80, and, more preferably, of 40-50 times the molar amount of the protected compound **3.**

The addition of the acid is carried out at a temperature of 20-35 °C, preferably of 30-35 °C. The resulting solution is then maintained under stirring at 30-40 °C for from a time of from 10 to 36 h, preferably for 25-30 h, by following the deprotection of the ligand e.g. by chromatography.

The acidic solution is then cooled e.g. at 25 °C, and then is neutralized by addition of a base, preferably NaOH, to achieve a raw solution with a final pH of from 6.5 to 7.5 which is then purified.

The purification steps preferably include: i) distillation of the neutralized solution, to remove the formed t-butanol, ii) desalination of the distillation residue, and iii) chromatographic purification of the desalinated solution.

In particular, in one embodiment, the solution resulting from the addition of a base is first distilled, preferably at a temperature of from 40 to 60 °C to remove formed t-butanol. The distillation residue is then desalinated, preferably by nanofiltration, and the collected solution is purified.

In one embodiment, the solution obtained by nanofiltration is first concentrated, for instance under vacuum at a temperature e.g. of 40 to 60 °C, preferably of about 50 °C, to a concentration preferably of 23-27% (w/w) and then is purified by elution on resins, more preferably on Amberlite XAD^{®} 1600. The eluate is optionally treated with Carbonpuron^{®} and concentrated under vacuum at about 50 °C to achieve an aqueous solution or aqueous mixture of the ligand of formula **4** with final concentration preferably ranging from 8-25%, that is used as such in the next complexation reaction, without any isolation of the ligand.

Advantageously, the above procedure comprises using water as the sole or one of the main reaction solvent, thus avoiding or reducing the use of organic solvents, and in particular of harsh solvents, such as DCM, and of harsh reactant, such as TFA and TIPS, which are required in the process of above-mentioned prior art. These harsh materials are difficult to handle, and are thus unsuitable for use in large-scale productions. Moreover, this step leads to achieve the desired ligand in an aqueous solution or aqueous mixture ready for use in the complexation reaction, without requiring its isolation.

### Step 5

The step comprises the complexation of the dimeric ligand of formula **4** with gadolinium ions to achieve an aqueous solution or aqueous mixture of the desired chelated complex **5.**

More particularly, the step preferably comprises:
i) Addition of a gadolinium salt, such as GdCl₃ to the solution of the ligand collected from step **4)** to obtain a mixture comprising the dimeric chelated complex **5;**
ii) Addition of a base to give a mixture with a pH value of from about 5 to about 7;
iii) Purification of the mixture, to give a solution of the dimeric complex of formula **5;** and
iv) Concentration of the collected solution.

The reaction is preferably carried out by addition of GdCl₃ directly to the solution of the ligand collected from the previous step of the process. The addition is preferably performed at a temperature of 25-45 °C. The required amount of GdCl₃ leading to achieve the exhaustive complexation of the ligand is determined by titration of the ligand solution, for instance by using copper sulfate as titrating agent, according to know procedures.

In one embodiment, the ratio between ligand of formula **4** and added GdCl₃ is from 1:1.98 to 1:2.02 (mol/mol); more preferably is of 1:2.00 to ensure the exhaustive consumption of the added lanthanide ion.

After the addition, the pH of the resulting mixture is adjusted to a value ranging from about 5 to about 7.5 by addition of a base, preferably NaOH.

For instance, in one embodiment GdCl₃ is added to the ligand solution e.g. at a temperature of 20-25 °C. The resulting mixture is adjusted to a pH value of 7-7.5, such as about 7 by addition of NaOH, and then is maintained under stirring at 20-25 °C for about 25 h, thus achieving an exhaustive complexation of the ligand.

In one alternative embodiment, GdCl₃ and the necessary amount of NaOH maintaining the pH at the desired neutral value can be added simultaneously, and the achieved mixture is then maintained under stirring for about 25 h, as above said.

In a preferred embodiment, the mixture resulting from the addition of GdCl₃ is adjusted to a pH value from about 5 to about 6, preferably from 5 to 5.6, more preferably of about 5.3, and then is maintained under stirring at about 40 °C for 1-4 h, e.g. about 2 h. The optional presence of residual free species such as free Gd³⁺, ligand or partially complexed ligand is then assessed, e.g. by titration and/or HPLC methods, and compensated by addition of calculated amounts of ligand or GdCl₃ to give an aqueous solution or aqueous mixture of the dimeric complex of formula **5** which is then purified.

The purification is preferably carried out by chromatography, preferably on resins.

In one embodiment the purification comprises the elution of the mixture resulting from the complexation reaction on a polymeric resin, preferably a Amberlite XAD^{®} 1600 resins.

In another embodiment, the purification comprises a first elution of the mixture resulting from the complexation reaction on a chelating resin, for instance selected from Hi Trap IMAC FF, Lewatit MonoPlus TP 260, Lewatit TP 208, IRC748I, DIAION CR11, SiliaMets AMPA and SiliaMets DOTA, and preferably from Diaion CR11 and Amberlite IRC748, allowing to minimize any optional free gadolinium content, and the additional purification of the collected eluate on a polymeric resin, such as a Amberlite XAD^{®} 1600 resin.

According to a practical implementation, a mixture adjusted to an about neutral pH value is properly purified by elution on Amberlite XAD^{®}1600 resin.

The mixtures resulting from regulation of the solution pH to lower values, such as 5-5.6, are otherwise preferably eluted first on a chelating resin such as the Amberlite IRC748 or the Diaion CR11 resin. The collected eluates are then preferably re-adjusted to a pH value of about 5.5-6 and concentrated, preferably under vacuum at 50 °C to obtain an aqueous solution or aqueous mixture of the dimeric complex with a concentration preferably of about 25% (w/w) that is then purified on Amberlite XAD^{®} 1600 resin.

Collected fractions are then optionally treated with charcoal and filtered. The resulting filtered solution is then preferably concentrated, for instance by distillation under vacuum at 45-55 °C to give a solution of the dimeric complex **5** with a final concentration of about 25% (w/w).

### Step 6

The dimeric complex of formula **5** is then isolated. The complex can be isolated from the aqueous solution or aqueous mixture from step **5)** for instance by lyophilization or by spray-drying. In one preferred embodiment the desired dimeric complex is obtained as a white solid by spray-drying the solution directly collected from step 5 of the process.

The overall yield of the process, determined from the limiting reactant as free base DO3A tri-tert-butyl ester **1A,** is of at least 20%, preferably of 25%, more preferably of about 28-30%, or even >30%.

Interestingly the above process comprises steps that are carried out one-pot, which are suitable for a large-scale implementation, and which do not require the isolation of any of the prepared precursor (such as the compound of formula **1A)** or reaction intermediates. As a result the synthetic approach object of the present invention allows to achieve the final product with an overall yield increased of at least about 18% over the process disclosed in WO2017098044.

In addition the lack of intermediates isolation allows for a reduction of the overall times process.

Moreover, the proposed process comprises the use of water, or more in general of aqueous solvent or aqueous solvent mixture, as the reaction solvent in all the steps following the preparation of the coupling product **3.** In particular, when the compound of formula **3** is prepared in step **3)** in an organic solvent, e.g. when it is prepared by reacting the compound of formula **2** from step **2)** with DO3A tri-tert-butyl ester **1A** from step **1),** the organic solvent can be replaced with an aqueous one or with an aqueous mixture by methods known to the skilled person, e.g. by first diluting with water, a water/organic solvent mixture, or an aqueous solution the organic solution of the compound of formula **3,** and then optionally by removing the organic solvent to obtain an aqueous solution of the compound of formula **3.** Using aqueous solvents or aqueous solvent mixtures as reaction solvent in all steps following the preparation of the coupling is very advantageous, particularly from the standpoint of costs, environmental impact, and ease of implementation in industrial scale. Indeed, the process disclosed in WO2017098044, uses solvent such as DCM and materials such as TFA and TIPS that beside being expensive are also difficult to handle, particularly when scaling the process on an industrial scale, and might also not be safe from a worker health point of view as well. As the process of the invention avoids or strongly reduces the use of organic solvents by using aqueous solvents or aqueous solvent mixtures in all the steps following the preparation of the compound of formula **3,** the problem of the prior art process is solved by the present invention because the latter is suitable, and can be easily implemented, for working on larger scales, for example for working in industrial processes. Moreover, the process of the invention surprisingly provides very high yields of the isolated dimeric complex, in particular yields that are higher than the ones of the prior art process, even though it comprises only using aqueous solvents or aqueous solvent mixtures following the preparation of the compound of formula **3.**

All solvents and starting materials, including reactants such as epichlorohydrin, D-glucamine, and the hydrobromide salt of DO3A tri-tert-butyl ester are commercially available, or can be obtained according to know procedures.

In a preferred embodiment, the hydrobromide salt of the DO3A tri-tert-butyl ester used as starting material for the preparation of a solution of the respective ester **1A** is prepared by using the manufacturing process described in the co-pending EP19215900.2 patent application (same applicant as the present application) and exemplified below, in the experimental section of the description, and stored until the use.

Non-limiting examples of preferred embodiments of the process of the invention are reported in the following section, aimed to illustrate the invention in greater detail without limiting its scope.

### EXPERIMENTAL PART

### Abbreviations and Definition of Terms

- DO3A tri-tert-butyl ester:: tri-tert-butyl 1,4,7,10-tetraazacyclododecane-1,4,7-triacetate
- DO3A tri-tert-butyl ester-HBr:: tri-tert-butyl 1,4,7,10-tetraazacyclododecane-1,4,7-triacetate hydrobromide salt
- TAZA:: 1,4,7,10-tetraazacyclododecane
- tBuOK: Potassium tert-butoxide
- DMAC: N,N-dimethylacetamide
- DMC: Dichloromethane
- DIPEA: N,N-diisopropylethylamine
- HCl: Hydrocloric acid
- MeCN: Acetonitrile
- NaOH: Sodium hydroxyde
- NH₃: Ammonia
- MRI: Magnetic Resonance Imaging
- MeCN: Acetonitrile
- NMM: N-methylmorpholine
- K₂CO₃: Potassium carbonate
- TFA: Trifluorocetic acid
- TIPS: triisopropylsilane
- FLD: Fluorescence detector
- UV/Vis: Unltraviolet/Visible

### HPLC characterization of the obtained compounds.

### General procedures

### Procedure 1: HPLC Characterization and determination of the assay of the DO3A-tri-tert-butyl ester

### Chromatographic conditions

| | | |
|---|---|---|
| HPLC system | Liquid chromatograph (*e.g*. Agilent 1100), equipped with solvent delivery system, autosampler, column thermostat, degasser and diode array detector or variable wavelength detector (or equivalent). | |
| Stationary phase: | Zorbax Eclipse XDB-C8, 5 µm, 150x4.6 mm | |
| Column temperature | 45 °C | |
| Mobile phase: | A: 0.01 M K₂HPO₄, 0.017 M H₃PO₄ | |
| | B: Acetonitrile | |
| Elution: Gradient | **Time (min)** | **%B** |
| | 0 | 5 |
| | 30 | 80 |
| | 35 | 80 |
| | 38 | 5 |
| | 45 | 5 |
| Flow | 1 mL/min | |
| Temperature | 45 °C | |
| Detection | UV, 210 nm, Bw=8 nm; Reference 360 nm, Bw=100 nm | |
| Injection volume | 10 µL | |
| Stop time | 35 min | |
| Reference peak | DO3A 3tBu | |
| Retention time | DO3A 3tBu ≅ 14-15 min. | |

**Procedure 2: HPLC method for monitoring the formation of Intermediate 2**

This method is employed for monitoring the mixture at the end of the alkylation of the D-glucamine and after distillation of the water.

### Chromatographic conditions

| | | |
|---|---|---|
| HPLC system: | Liquid chromatograph (e.g. Agilent 1100), equipped with solvent delivery system, autosampler, column thermostat, degasser and diode array detector or variable wavelength detector (or equivalent) | |
| Stationary phase: | SeQuant ZIC-cHilic 3 µm, 150x2.1 mm (Merck P.N. 1.50658.0001) | |
| Column Temperature: | 40 °C | |
| Mobile phase: | gradient elution | |
| | Eluent A = 5 mM ammonium acetate | |
| | Eluent B = ACN/MeOH, 75/25 | |
| Elution: Gradient | **Time (min)** | **%B** |
| | 0 | 97 |
| | 5 | 97 |
| | 30 | 20 |
| | 40 | 20 |
| | 45 | 97 |
| | 60 | 97 |
| Flow rate: | 0.25 mL/min | |
| Detection: | UV, 210-240 nm | |
| Injection volume: | 10 µL | |
| Run time: | 60 min | |
| Dilution solution | ACN/MeOH, 75/25 | |
| Sample preparation: | add 200 µL of 5 mM ammonium acetate solution to 75 µL of mixture and dilute to 5 mL with dilution solution. | |

### Procedure 3: HPLC method for monitoring the formation and the purification of Intermediate 3

### General procedure

The method is used for monitoring the formation of the Intermediate **3** and the purification step.

### Analytical conditions

| | | |
|---|---|---|
| HPLC system | Liquid chromatograph Agilent 1100 | |
| Stationary phase: | Gemini, 5 µm, 250x4.6 mm (Phenomenex, item 00G-4435-EO) 40 °C | |
| Column temperature | | |
| Mobile phase: | A: mobile phase A | |
| | B: MeCN | |
| Elution: Gradient | **Time (min)** | **%B** |
| | 0 | 40 |
| | 5 | 40 |
| | 30 | 90 |
| | 35 | 90 |
| | 36 | 40 |
| | 45 | 40 |
| Flow | 0.7 mL/min | |
| Detection | UV/210 nm | |
| Injection volume | 10 µL | |
| Stop time | 45 min | |
| INT 2 Rₜ | 21 min | |

### Mobile phase A

### Preparation of the solution

In a 1000 mL volumetric flask accurately weigh 2.0 g of ammonium acetate and then dilute to volume with water. In a 1000 mL volumetric flask transfer 600 mL of ammonium acetate solution and 300 mL of methanol. Sonicate for half an hour.

### Procedure 4: HPLC method for monitoring the formation and purification of the chelating ligand 4.

### General procedure

The monitoring of the formation and purification of the dimeric ligand **4** were performed by reverse-phase HPLC with UV detection at 210 nm.

### Analytical conditions

| | | |
|---|---|---|
| HPLC system | Liquid chromatograph Agilent 1260 Infinity | |
| Stationary phase: | Synergi Polar-RP, 4 µm, 150x4.6 mm (Phenomenex, item 00F-4336-EO) | |
| Column temperature | 40 °C | |
| Mobile phase: | A: 10 mM KH₂PO₄ | |
| | B: Methanol | |
| Elution: Gradient | **Time (min)** | **%B** |
| | 0 | 0 |
| | 5 | 0 |
| | 35 | 60 |
| | 40 | 60 |
| | 41 | 80 |
| | 46 | 80 |
| | 47 | 0 |
| | 60 | 0 |
| Flow | 0.8 mL/min | |
| Detection | UV/210 nm | |
| Injection volume | 10 µL | |
| Stop time | 60 min | |
| Compound 4 Rₜ | 2.4 min | |

### Example 1: synthesis of DO3A-tri-tert-butyl ester hydrobromide salt

The synthesis of the starting DO3A tri-tert-butyl ester hydrobromide salt was performed by using the procedure constituting the object of the co-pending patent application EP19215900.2 (same applicant as the present application). In particular:
To a suspension of commercially available TAZA (14.39 kg; 83.53 mol) and sodium acetate (21.58 kg; 263.12mol) in DMAC (98.07 kg; 104.33 L), a solution of tert-butyl bromoacetate (51.32kg; 263.12 mol) in DMAC (50.72 kg; 53.96 L) was added at 10 °C during 2.5 h. Then the temperature was raised to 25 °C and the mixture was stirred for 24 h at this temperature. Water (57.56 kg) was then added in 0.5 h and after 2 h the mixture was centrifuged and washed with water (2x57 kg). The wet solid was dried under vacuum obtaining 36,62 kg; 61.48 mol of DO3A tri-tert-butyl ester hydrobromide (73.6% yield). The assay determined by HPLC (against standard) of the product is 100% w/w; the assay determined by NMR (against standard) is 99.86% w/w.

### Example 2: preparation of the dimeric compound 5

The dimeric complex compound **5** is obtained by using the synthetic procedure schematized below 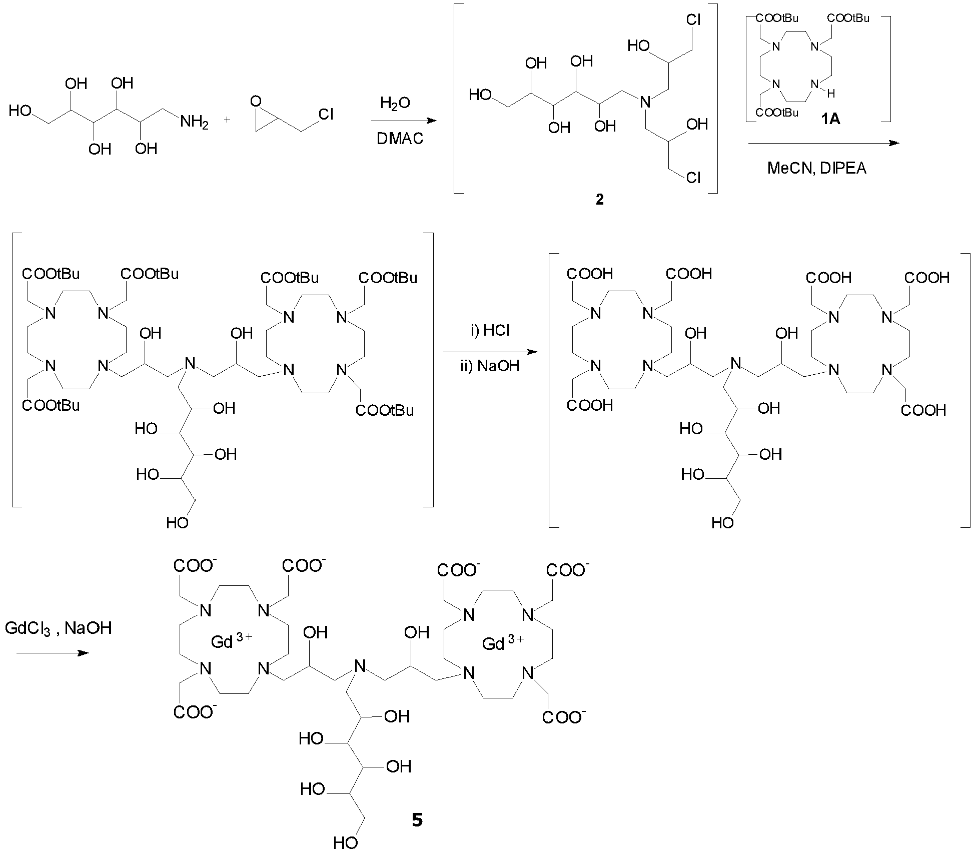 comprising:
a) Neutralization of the hydrobromide to give DO3A tri-tert-butyl ester **1A**
   DO3A tri-tert-butyl ester-HBr obtained as described in Example 1 (36.62 kg; 61.5 mol) and potassium carbonate (16.99 kg; 122.9 mol) were suspended in MeCN (72.29 kg; 91.50 L) at room temperature and the mixture was kept under stirring for 20 h at 24 °C. The resulting salts were then filtered off, and the filtrate was partially distilled at 50 °C under vacuum to give a solution of DO3A tri-tert-butyl ester **1A** (30.68 kg; 59.6 mol) in MeCN with a final concentration of about 62% w/w that was used in the next step without further isolation. The **1A** assay in solution was determined by an HPLC-UV method.
b) Synthesis of the Intermediate **2**
   D-glucamine (6 kg; 33.1 mol) in water (15 kg) was dropped in a solution of epichlorohydrin (6.74 kg; 72.8 mol) in DMAC (6 kg; 6.38 L) at room temperature in 2 h. The mixture was kept under stirring for 17 h. Then the mixture was diluted with DMAC (12 kg; 12.77 L) and the water was distilled at 45-50 °C under vacuum to achieve a solution of the intermediate **2** in DMAC with a residual water content < 2.0% that was used for the next step without further purification.
c) Alkylation of the intermediate **2** with DO3A tri-tert-butyl ester **1A** to give the protected ligand **3**
   DIPEA (9.79 kg; 75.8 mol) and the solution of substrate **1A** collected from step a) were added to the solution of intermediate **2** heated at 50 °C and the obtained mixture was then stirred at 70 °C for 72 h by monitoring the conversion by HPLC-UV method. The mixture was then cooled to 20÷25 °C, diluted with 16% w/w ammonia aqueous solution (36 L). The resulting mixture was kept under stirring at 25 °C for 15 h obtaining precipitation of salts that were removed by filtration. The filtrate was then purified by chromatography on Amberlite XAD^{®} 1600 (450 L; eluent: gradient of water/MeCN). Then pure fractions (HPLC Area % ≥ 90) were collected, the organic solvent was distilled, and the aqueous residue was concentrated at about 50 °C under vacuum to give a solution of the protected ligand **3** in water (conc. of about 10% w/w) suitable for use as such in the next step without any further isolation.
d) Deprotection
   34% hydrochloric acid aqueous solution (64.72 kg; 603.5 mol) was added to the solution of the protected ligand **3** collected from step c) by maintaining the temperature at about 30 °C. At the end of the addition, the mixture was heated to 35÷40 °C and kept under stirring for 30 h. The solution was cooled to 20÷25 °C and neutralized (final pH about 7) by addition of 30% sodium hydroxide aqueous solution, the t-butanol formed as the reaction by-product was removed by distillation and the mixture was desalinated by nanofiltration. The desalinated mixture was then partially concentrated at 50 °C under vacuum to a final concentration of 25% (w/w) and purified by chromatography on Amberlite XAD^{®}1600 (150 L; eluent water). Pure fractions (HPLC Area % ≥ 90) were treated with charcoal and concentrated at 45-55 °C under vacuum to obtain a 10% (w/w) water solution of the desired ligand **4** (11.4 mol), which was quantified by potentiometric titration using a copper sulfate solution as titrating agent.
e) Complexation

The solution of ligand **4** was heated to 40 °C, and added with an aqueous solution of gadolinium chloride (21.35 kg of solution; 6.01 kg of gadolinium chloride; 22.8 mol) maintaining the temperature around 40 °C. After addition, the pH of the solution was adjusted to 5.3 by addition of 10% sodium hydroxide in water and the mixture was maintained at 40 °C for 2 h with formation of the respective paramagnetic complex **5.** The presence of any free species was assessed e.g. by titration. A final solution of the dimeric complex is thus obtained that was purified on Diaion CR11 (6 L) reducing any free gadolinium content. After loading, the resin was washed with water; the collected solution was adjusted to a pH of 5.5 and then concentrated under vacuum at 50 °C to give a 25% w/w water solution that was then loaded at pH 6.2 on Amberlite XAD^{®}1600 (500 L; eluent: gradient of water/MeCN). The selected fractions were treated with charcoal and then distilled at 50 °C under vacuum to obtain a mixture with a final concentration of about 25% (w/w) from the which the gadolinium complex is isolated by spray-drying as a white powder (11.74 kg; 10.80 kg on anhydrous base; 8.3 mol) with a final titration assay of 99%, (w/w %; on anhydrous base).
Overall yield determined from DO3A tri-tert-butyl ester **1A:** 28%.

### Example 3: preparation of Compound 5

### a) Neutralization of DO3A 3tBu-HBr

DO3A tri-tert-butyl ester-HBr (241.6 g; 0.406 mol) and potassium carbonate (112.1 g; 0.811 mol) were suspended in MeCN (477.2 g; 0.604 L) and the mixture was kept under stirring for 20 h at 25 °C. The formed salts were then filtered off, the filtrate was partially distilled at 50 °C under vacuum to give a solution of DO3A tri-tert-butyl ester **1A** (204.5 g; 0.397 mol) in MeCN with a final concentration of 61% w/w that was used in the next step without further isolation. The **1A** assay in solution was determined by an HPLC-UV method.

### b) Synthesis of intermediate 2

D-glucamine (40.0 g; 0.221 mol) in water (99.3 g) was dropped in a solution of epichlorohydrin (44.9 g; 0.486 mol) in DMAC (40.0 g; 0.043 L) at 24 °C in 2 h. The mixture was kept under stirring for 17 h, then DMAC was added (80.0 g; 0.085 L) and the water was distilled at 50 °C under vacuum to achieve a solution of the intermediate 2 in DMAC with a residual water content < 2.0% w/w.

### c) Alkylation of compound 1A with Intermediate 2

DIPEA (65.3 g; 0.505 mol) and the solution of **1A** were added to the solution of the Intermediate **2** collected from step b) at 50 °C. The mixture was further stirred at 70 °C for 72 h by monitoring the conversion by HPLC-UV method, then, after cooling to 25 °C, it was diluted with 16% w/w ammonia aqueous solution (0.240 L). The resulting mixture was kept under stirring at 20 °C for 15 h obtaining precipitation of salts that were removed by filtration. The filtrate was then purified by chromatography on Amberlite XAD^{®} 1600 (3 L; eluent: gradient of water/MeCN). Pure fractions (HPLC Area % ≥ 90) were collected and distilled to remove the organic solvent. The resulting residue was then concentrated at 50 °C under vacuum to give a solution of the protected ligand **3** in water with a final concentration of about 10% w/w that was used as such in the next step of the process.

### d) Deprotection of Intermediate 3

34% hydrochloric acid aqueous solution (431.5 g; 4.023 mol) was added to the solution of Intermediate **3** from step **c)** by maintaining the temperature at 30÷35 °C. At the end of the addition, the mixture was heated to 37 °C and kept under stirring for 36 h. Then the solution was cooled to 25 °C and neutralized by addition of 30% sodium hydroxide aqueous solution, the t-butanol formed as by-product was removed by distillation and the mixture was desalinated by nanofiltration. The mixture was then partially concentrated at 50 °C under vacuum up to concentration of 24% w/w and purified by chromatography on Amberlite XAD^{®}1600 (1L; eluent water). The fractions selected by evaluation on HPLC-UV were treated with charcoal and concentrated at 50 °C under vacuum to obtain a 10% w/w water solution of the desired ligand **4** (0.075 mol), which was quantified by potentiometric titration using a copper sulfate solution as titrating agent.

### e) Complexation

The solution of the deprotected ligand **4** was heated to 37 °C, then gadolinium chloride aqueous solution (140.5 g of solution; 39.5 g of gadolinium chloride; 0.150 mol) was added maintaining the temperature in the range 37÷43 °C. At the end of the addition, the pH was adjusted to 5.3 by addition of 10% sodium hydroxide aqueous solution. The mixture was maintained at 40 °C for 2 h with formation of the respective paramagnetic complex **5.** The presence of any free species was assessed e.g. by titration. The solution was then purified on Diaion CR11 chelating resin (0.16 L) reducing any free gadolinium content. After loading, the resin was washed with water, the pH adjusted to 5.5 and the solution concentrated under vacuum at 50 °C to obtain a 25% w/w water solution. This solution was loaded at pH 6 on Amberlite XAD^{®}1600 (3.3 L; eluent: gradient of water/MeCN). The fractions selected by evaluation on HPLC-FLD and UV were treated with charcoal and the resulting solution distilled at 50 °C under vacuum. The final solution (25% w/w) was spray-dried to isolate the gadolinium complex as a white powder (82.0 g corresponding to 74.6 g, as anhydrous product; titration assay: 99% w/w %, anhydrous base).

Overall yield from DO3A tri-tert-butyl ester **1A:** 29%.

### Example 4: preparation of Compound 5

### a) Neutralization of DO3A 3tBu-HBr

DO3A 3tBu-HBr (241.6 g; 0.406 mol) and potassium carbonate (112.1 g; 0.811 mol) were suspended in MeCN (480.0 g; 0.608 L) and the mixture was kept under stirring for 18 h at 27 °C. The formed salts were then filtered off, the filtrate was partially distilled at 50 °C under vacuum to give a solution of DO3A tri-tert-butyl ester **1A** (205.0 g; 0.398 mol) in MeCN with a final concentration of 58% w/w that was used in the next step without further isolation. The **1A** assay in solution was determined by an HPLC-UV method.

### b) Synthesis of Intermediate 2

D-glucamine (40.0 g; 0.221 mol) in water (100.0 g) was dropped in a solution of epichlorohydrin (44.9 g; 0.486 mol) in DMAC (40.0 g; 0.043 L) at 25 °C in 2 h. The mixture was kept under stirring for 16 h, then DMAC was added (80.0 g; 0.085 L) and the water was distilled at 50 °C under vacuum to achieve a solution of the intermediate 2 in DMAC with a residual water content < 2.0% w/w.

### c) Alkylation of compound 1A with Intermediate 2

DIPEA (65.3 g; 0.505 mol) and the solution of **1A** were added to the solution of the Intermediate **2** collected from step b) at 50 °C. The mixture was further stirred at 75 °C for 70 h by monitoring the conversion by HPLC-UV method, then was partially concentrated at 60 °C under vacuum. After cooling to 23 °C, a mixture of water (80.0 g) and MeCN (126.4 g; 0.160 L), previously prepared, and after 25% w/w ammonia aqueous solution (144.5 g; 0.160 L) were added. The resulting mixture was kept under stirring at 22 °C for 14 h. The mixture was filtered and purified by chromatography on Amberlite XAD^{®} 1600 (3 L; eluent: gradient of water/MeCN). After elution the fractions with adequate purity (HPLC Area % ≥ 90) were collected, the organic solvent distilled, and the resulting solution concentrated at 50 °C under vacuum to give the protected ligand **3** in water solution (concentration: 13% w/w) which is used as such in the next step.

### d) Deprotection of Intermediate 3

34% hydrochloric acid aqueous solution (435.0 g; 4.057 mol) was added to the solution of Intermediate **3** from step c) by maintaining the temperature at 30÷35 °C. At the end of the addition, the mixture was kept at 35 °C under stirring for 32 h. After, the solution was cooled to 23 °C and neutralized by addition of 30% sodium hydroxide aqueous solution, the t-butanol formed as by-product was removed by distillation and the mixture was desalinated by nanofiltration. The mixture was then partially concentrated at 50 °C under vacuum up to concentration of 22% w/w and purified by chromatography on Amberlite XAD^{®}1600 (1 L; eluent water). The fractions selected by evaluation with HPLC-UV were treated with charcoal and concentrated at 50 °C under vacuum to obtain a 25% w/w water solution of the desired ligand **4** (0.076 mol), which was quantified by potentiometric titration using a copper sulfate solution as titrating agent.

### e) Complexation

To the solution of deprotected ligand **4** at 25 °C, a gadolinium chloride aqueous solution (142.4 g of solution; 40.1 g of gadolinium chloride; 0.152 mol) was added maintaining the temperature in the range 23÷27°C adjusting the pH to 7.0÷7.5 by addition of 30% sodium hydroxide aqueous solution. The mixture was maintained at 25 °C for 24 h with formation of the respective paramagnetic complex **5.** The presence of any free species was assessed e.g. by titration. The resulting solution was purified on Amberlite XAD^{®}1600 (3.3 L; eluent: gradient of water/MeCN). The fractions selected by evaluation on HPLC-FLD and UV were treated with charcoal and the resulting solution distilled at 50 °C under vacuum. The final solution (25% w/w) was spray-dried to isolate the gadolinium complex as a white powder (73.5 g corresponding to 66.9 g, as anhydrous product; titration assay: 99% w/w %, anhydrous base).

Overall yield from DO3A tri-tert-butyl ester **1A:** 26%.

### Example 5: preparation of Compound 5

### a) Neutralization of DO3A 3tBu-HBr

DO3A 3tBu-HBr (724.9 g; 1.217 mol) and potassium carbonate (336.4 g; 2.434 mol) were suspended in MeCN (1431.7 g; 1.812 L) and the mixture was kept under stirring for 18 h at 25 °C. The formed salts were then filtered off, the solution was partially distilled at 50 °C under vacuum to give a solution of DO3A tri-tert-butyl ester **1A** (613.9 g; 1.193 mol) in MeCN with a final concentration of 56% w/w that was used in the next step without further isolation. The **1A** assay in solution was determined by an HPLC-UV method.

### b) Synthesis of Intermediate 2

D-glucamine (120.0 g; 0.662 mol) in water (298.0 g) was dropped in a solution of epichlorohydrin (134.8 g; 1.457 mol) in DMAC (120.0 g; 0.128 L) at 24 °C in 2 h. The mixture was kept under stirring for 18 h, then DMAC was added (240.0 g; 0.255 L) and the water was distilled at 50 °C under vacuum to achieve a solution of the intermediate **2** in DMAC with a residual water content < 2.0% w/w.

### c) Alkylation of compound 1A with Intermediate 2

DIPEA (195.9 g; 1.516 mol) and the solution of substrate **1A** were added to the solution of the intermediate **2** at 45 °C. The mixture was then maintained under stirring at 70 °C for 75 h, by monitoring the conversion by HPLC-UV method. The mixture was then partially concentrated at 60 °C under vacuum. The residue was cooled to 25 °C, diluted with a mixture of water (720.0 g) and MeCN (379.2 g; 0.480 L) previously prepared. The resulting solution was then purified by chromatography on Amberlite XAD^{®} 1600 (9 L; eluent: gradient of water/MeCN). The fractions with adequate purity (HPLC Area % ≥ 90) were collected and the obtained solution was distilled to remove the organic solvent. An aqueous residue is thus obtained that is concentrated at 50 °C under vacuum to give a solution of the protected ligand **3** in water with a final concentration of 15% w/w, which is used for the next step.

### d) Deprotection of Intermediate 3

34% hydrochloric acid aqueous solution (1294.4 g; 12.070 mol) was added to the collected solution of Intermediate **3,** by maintaining the temperature at 30÷35 °C. At the end of the addition, the mixture was heated to about 40 °C and kept under stirring to this temperature for 28 h. Then the solution was cooled to 25 °C and neutralized by addition of 30% sodium hydroxide aqueous solution. The formed t-butanol was removed by distillation and the residual mixture was first desalted by nanofiltration and then partially concentrated at 50 °C under vacuum up to concentration of 26% w/w. The concentrated solution was then purified by chromatography on Amberlite XAD^{®}1600 (3 L; eluent water). Pure fractions were collected, treated with charcoal and concentrated at 50 °C under vacuum to obtain a 12% w/w water solution of the deprotected ligand **4** (0,228 mol), which was quantified by potentiometric titration using a copper sulfate solution as titrating agent.

### e) Complexation

The solution of deprotected ligand **4** was heated to 37 °C, then gadolinium chloride aqueous solution (427.1 g of solution; 120.2 g of gadolinium chloride; 0.456 mol) was added maintaining the temperature in the range 37÷43 °C. At the end of the addition, the pH was adjusted to 5.5 by addition of 10% sodium hydroxide aqueous solution and the obtained mixture was maintained at 42 °C for 2 h with formation of the respective paramagnetic complex **5.** The presence of any free species was assessed e.g. by titration. The solution was purified at 25 °C on Diaion CR11 chelating resin (0.48 L) reducing any free gadolinium content. After loading, the resin was washed with water, the pH adjusted to 5.8 and the solution concentrated under vacuum at 50 °C to obtain a 26% w/w water solution. This solution was loaded at pH 6.2 on Amberlite XAD^{®}1600 (10 L; eluent: gradient of water/MeCN). The fractions selected by evaluation on HPLC-FLD and UV were treated with charcoal and the resulting solution distilled at 50 °C under vacuum. The final solution (22% w/w) was spray-dried to isolate the gadolinium complex as a white powder (226.5 g corresponding to 208.3 g, as anhydrous product; titration assay: 99% w/w %, anhydrous base).

Overall yield from DO3A tri-tert-butyl ester **1A:** 27%.

### Example 6: preparation of Compound 5

### a) Neutralization of DO3A 3tBu-HBr

DO3A 3tBu-HBr (724.9 g; 1.217 mol) and potassium carbonate (336.4 g; 2.434 mol) were suspended in MeCN (1435.5 g; 1.817 L) and the mixture was kept under stirring for 18 h at 25 °C. The formed salts were then filtered off, the solution was partially distilled at 50 °C under vacuum to give a solution of DO3A tri-tert-butyl ester **1A** (607.9 g; 1.181 mol) in MeCN with a final concentration of 62% w/w that was used in the next step without further isolation. The **1A** assay in solution was determined by an HPLC-UV method.

### b) Synthesis of Intermediate 2

D-glucamine (120.0 g; 0.662 mol) in water (300.0 g) was dropped in a solution of epichlorohydrin (134.8 g; 1.457 mol) in DMAC (120.0 g; 0.128 L) at 25 °C in 2 h. The mixture was kept under stirring for 18 h, then DMAC was added (240.0 g; 0.255 L) and the water was distilled at 50 °C under vacuum to achieve a solution of the intermediate 2 in DMAC with a residual water content < 2.0% w/w.

### c) Alkylation of compound 1A with Intermediate 2

DIPEA (196.1 g; 1.517 mol) and the solution of substrate **1A** were added to the solution of **2** at 50 °C. The mixture was then maintained under stirring at 70 °C for 70 h, by monitoring the conversion by HPLC-UV method, then, after cooling to 25 °C, it was diluted with water (720.0 g) The resulting mixture was then purified by chromatography on Amberlite XAD^{®} 1600 (9 L; eluent: gradient of water/MeCN). After elution the fractions with adequate purity (HPLC Area % ≥ 90) were collected, the organic solvent distilled and the resulting solution concentrated at 50 °C under vacuum to give a solution of the protected ligand **3** in water with a final concentration of 14% w/w, which is used for the next step.

### d) Deprotection of Intermediate 3

34% hydrochloric acid aqueous solution (1290.0 g; 12.030 mol) was added to a solution of Intermediate **3,** maintaining the temperature at 30÷35 °C. At the end of the addition, the mixture was heated to 38 °C and kept under stirring for 30 h. Then the solution was cooled to 25 °C and neutralized by addition of 30% sodium hydroxide aqueous solution. The formed t-butanol was removed by distillation and the residual mixture was first desalted by nanofiltration and then partially concentrated at 50 °C under vacuum up to concentration of 22% w/w. The concentrated solution was then purified by chromatography on Amberlite XAD^{®}1600 (3 L; eluent water). The fractions selected by evaluation on HPLC-UV were treated with charcoal and concentrated at 50 °C under vacuum to obtain a 23% w/w water solution of the desired ligand **4** (0,226 mol), which was quantified by potentiometric titration using a copper sulfate solution as titrating agent.

### d) Complexation

To the solution of the deprotected ligand **4** at 25 °C, a gadolinium chloride aqueous solution (423.3 g of solution; 119.2 g of gadolinium chloride; 0.452 mol) was added maintaining the temperature in the range 23÷27 °C adjusting the pH to 7.0÷7.5 by addition of 30% sodium hydroxide aqueous solution. The mixture was maintained at 25 °C for 24 h. The resulting solution was purified on Amberlite XAD^{®}1600 (10 L; eluent: gradient of water/MeCN). The fractions selected by evaluation on HPLC-FLD and UV were treated with charcoal and the resulting solution distilled at 50 °C under vacuum. The final solution (25% w/w) was spray-dried to isolate the gadolinium complex as a white powder (201.3 g corresponding to 185.2 g, as anhydrous product; titration assay: 99% w/w %, anhydrous base).

Overall yield from DO3A tri-tert-butyl ester **1A:** 24%.

### Example 7: preparation of Compound 5

### a) Neutralization of compound 1.

DO3A 3tBu-HBr 1 (724.9 g; 1.217 mol) and potassium carbonate (336.4 g; 2.434 mol) were then suspended in MeCN (1431.7 g; 1.812 L) and the mixture was kept under stirring for 18 h at 25 °C. The salts were then filtered off, the solution was partially distilled at 50 °C under vacuum and compound 1A (613.6 g; 1.192 mol, 98% conversion) was kept in MeCN solution (final concentration: 56% w/w) until the next step without further isolation.

### b) Synthesis of Intermediate 2.

D-glucamine (120.0 g; 0.662 mol) in water (298.0 g) was dropped in a solution of epichlorohydrin (134.8 g; 1.457 mol) in DMAC (120.0 g; 0.128 L) at 24 °C in 2 h. The mixture was kept under stirring for 18 h, then DMAC was added (240.0 g; 0.255 L) and the water was distilled at 50 °C under vacuum to achieve a solution of the intermediate **2** in DMAC with a residual water content < 2.0% w/w.

### c) Alkylation of compound 1A with Intermediate 2

DIPEA (195.9 g; 1.516 mol) and the solution of substrate 1A were added to the solution of the intermediate **2** at 50 °C. The mixture was then maintained under stirring at 70 °C for 75 h, by monitoring the conversion by HPLC-UV method (HPLC Area %: 75). The mixture was then partially concentrated at 60 °C under vacuum. The residue was cooled to 25°C, diluted with a mixture of water (240.0 g) and MeCN (379.2 g; 0.480 L) previously prepared, and then with 25% ammonia aqueous solution (433.4 g; 0.480 L) to give a mixture that was kept under stirring for 16 h, by obtaining the precipitation of hydrochloride salts that were filtered. The filtrate was then collected and purified by chromatography on Amberlite XAD^{®} 1600 (9 L; eluent: gradient of water/MeCN). The fractions with adequate purity (HPLC A% ≥ 90) were collected and the obtained solution was distilled to remove the organic solvent. An aqueous residue is thus obtained that is concentrated at 50 °C under vacuum to give a solution of the protected ligand 3 in water with a final concentration of 14% w/w, which is used for the next step.

### d) Deprotection of Intermediate 3.

34% hydrochloric acid aqueous solution (1294.4 g; 12.070 mol) was added to the collected solution of Intermediate **3,** by maintaining the temperature at 30÷35°C. At the end of the addition, the mixture was heated to about 40 °C and kept under stirring to this temperature for 30 h. Then the solution was cooled to 25°C and neutralized by addition of 30% sodium hydroxide aqueous solution. The formed t-butanol was removed by distillation and the residual mixture was first desalted by nanofiltration and then partially concentrated at 50 °C under vacuum up to concentration of 26% w/w. The concentrated solution was then purified by chromatography on Amberlite XAD^{®}1600 (3 L; eluent water). Pure fractions were collected, treated with charcoal and concentrated at 50 °C under vacuum to obtain a 25% w/w water solution of the deprotected ligand **4,** which was quantified by potentiometric titration using a copper sulfate solution as titrating agent.

### e) Complexation

The solution of deprotected ligand **4** was heated to 37°C, then gadolinium chloride aqueous solution (427.1 g; 120.2 g of gadolinium chloride; 0.456 mol) was added maintaining the temperature in the range 37÷43 °C. At the end of the addition, the pH was adjusted to 5.5 by addition of 10% sodium hydroxide aqueous solution and the obtained mixture was maintained at 42 °C for 2 h. The solution was purified at 25 °C on Diaion CR11 chelating resin (0.48 L) reducing any free gadolinium content. After elution, the resin was washed with water, the pH adjusted to 5.8 and the solution concentrated under vacuum at 50 °C to obtain a 26% w/w water solution. This solution was loaded at pH 6.2 on Amberlite XAD^{®}1600 (10 L; eluent: gradient of water/MeCN). The fractions selected by evaluation on HPLC-FLD and UV were treated with charcoal and the resulting solution distilled at 50 °C under vacuum. The final solution (22% w/w) was spray-dried to isolate the gadolinium complex as a white powder (251.6 g corresponding to 231.5 g, as anhydrous product; titration assay: 99% w/w%, anhydrous base).

Overall yield from DO3A tri-tert-butyl ester **1A:** 30%.

### Example 8: preparation of the dimeric compound 5

### a) Neutralization of the hydrobromide to give DO3A tri-tert-butyl ester 1A

DO3A tri-tert-butyl ester-HBr obtained as described in Example 1 (36.60 kg; 61.4 mol) and potassium carbonate (16.99 kg; 122.9 mol) were suspended in MeCN (72.50 kg; 91.77 L) at room temperature (i.e. 25 °C) and the mixture was kept under stirring for 19 h at 24 °C. The resulting salts were then filtered off, and the filtrate was partially distilled at 50 °C under vacuum to give a solution of DO3A tri-tert-butyl ester **1A** (31.07 kg; 60.4 mol) in MeCN with a final concentration of about 56% w/w that was used in the next step without further isolation. The **1A** assay in solution was determined by an HPLC-UV method.

### b) Synthesis of the Intermediate 2

D-glucamine (6 kg; 33.1 mol) in water (15.1 kg) was dropped in a solution of epichlorohydrin (6.75 kg; 73.0 mol) in DMAC (6.2 kg; 6.60 L) at room temperature in 2 h. The mixture was kept under stirring for 16 h. Then the mixture was diluted with DMAC (12.2 kg; 12.98 L) and the water was distilled at 55-60 °C under vacuum to achieve a solution of the intermediate **2** in DMAC with a residual water content < 2.0% that was used for the next step without further purification.

### c) Alkylation of the intermediate 2 with DO3A tri-tert-butyl ester 1A to give the protected ligand 3

DIPEA (9.80 kg; 75.8 mol) and the solution of substrate **1A** collected from step a) were added to the solution of intermediate **2** heated at 50 °C and the obtained mixture was then stirred at 70 °C for 80 h by monitoring the conversion by HPLC-UV method. The mixture was then partially concentrated at 60 °C under vacuum. The residue was cooled to 25°C, diluted with a mixture of water (12.2 kg) and MeCN (19.0 kg; 24.05 L) previously prepared, and then with 25% ammonia aqueous solution (21.7 kg; 24.03 L) to give a mixture that was kept under stirring for 15 h, by obtaining the precipitation of hydrochloride salts that were filtered. The filtrate was then collected and purified by chromatography on Amberlite XAD^{®} 1600 (450 L; eluent: gradient of water/MeCN). Then pure fractions (HPLC Area % ≥ 90) were collected, the organic solvent was distilled, and the aqueous residue was concentrated at about 50 °C under vacuum to give a solution of the protected ligand **3** in water (conc. of about 10% w/w) suitable for use as such in the next step without any further isolation.

### d) Deprotection

34% hydrochloric acid aqueous solution (64.75 kg; 603.8 mol) was added to the solution of the protected ligand **3** collected from step c) by maintaining the temperature at about 30 °C. At the end of the addition, the mixture was heated to 35÷40 °C and kept under stirring for 30 h. The solution was cooled to 20÷25 °C and neutralized (final pH about 7) by addition of 30% sodium hydroxide aqueous solution, the t-butanol formed as the reaction by-product was removed by distillation and the mixture was desalinated by nanofiltration. The desalinated mixture was then partially concentrated at 50 °C under vacuum to a final concentration of 25% (w/w) and purified by chromatography on Amberlite XAD^{®}1600 (150 L; eluent water). Pure fractions (HPLC Area % ≥ 90) were treated with charcoal and concentrated at 45-55 °C under vacuum to obtain a 12% (w/w) water solution of the desired ligand **4** (15.3 mol), which was quantified by potentiometric titration using a copper sulfate solution as titrating agent.

### e) Complexation

The solution of ligand **4** was heated to 40 °C, and added with an aqueous solution of gadolinium chloride (27.79 kg of solution; 8.10 kg of gadolinium chloride; 30.7 mol) maintaining the temperature around 40 °C. After addition, the pH of the solution was adjusted to 5.5 by addition of 10% sodium hydroxide in water and the mixture was maintained at 40 °C for 2 h with formation of the respective paramagnetic complex **5.** The presence of any free species was assessed e.g. by titration. A final solution of the dimeric complex is thus obtained that was purified on Diaion CR11 (6 L) reducing any free gadolinium content. After loading, the resin was washed with water; the collected solution was adjusted to a pH of 5.5 and then concentrated under vacuum at 50 °C to give a 23% w/w water solution that was then loaded at pH 5.7 on Amberlite XAD^{®}1600 (500 L; eluent: gradient of water/MeCN). The selected fractions were treated with charcoal and then distilled at 50 °C under vacuum to obtain a mixture with a final concentration of about 28% (w/w) from the which the gadolinium complex is isolated by spray-drying as a white powder (13.38 kg; 12.58 kg on anhydrous base; 9.7 mol) with a final titration assay of 99%, (w/w %; on anhydrous base).
Overall yield determined from DO3A tri-tert-butyl ester **1A:** 33%.

## Claims

1. A process for the manufacturing of the dimeric complex compound **5** of formula 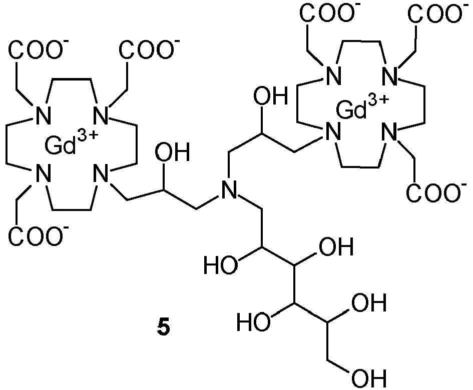 which comprises:
**1)** preparing a solution of DO3A tri-tert-butyl ester of formula **1A** 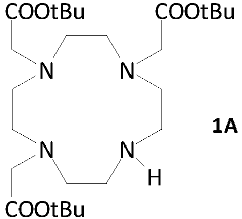 in an organic solvent;
**2)** preparing a solution of a compound of formula **2** 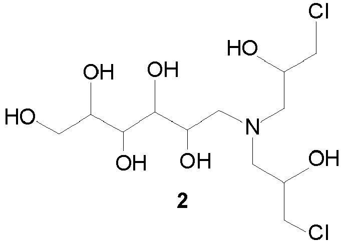 in an organic solvent;
**3)** admixing the solutions prepared according to steps **1)** and **2)** to obtain a solution of a compound of formula **3** 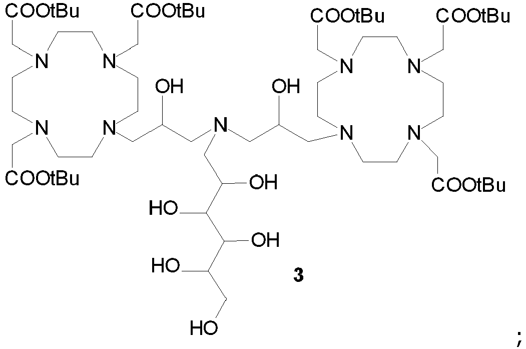
**4)** without isolating the compound from the solution of step **3),** removing the tert-butyl protecting groups from the compound of formula **3** to obtain a solution of a respective free ligand of formula **4** 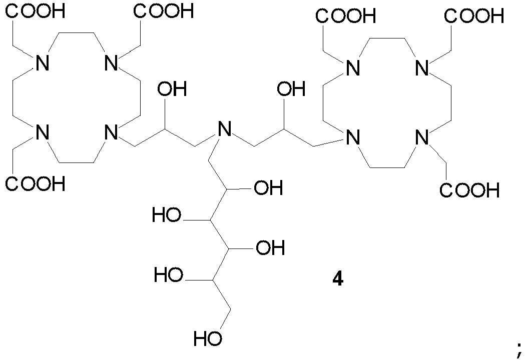
**5)** without isolating the free ligand of formula of formula **4,** adding gadolinium metal ions to the solution of step **4)** to obtain a solution of the respective dimeric complex of formula **5**; and
**6)** isolating the dimeric complex,
wherein the reaction solvent in all the steps following the preparation of the compound of formula **3** is an aqueous solvent.

2. The process of claim 1 wherein the solution of the DO3A tri-tert-butyl ester **1A** of step **1)** is prepared from a hydrobromide salt of the DO3A tri-tert-butyl ester.

3. The process of claims 1 or 2 wherein step **1)** comprises:
i) suspending a hydrobromide salt of the DO3A tri-tert-butyl ester together with a base or a basic salt in an organic solvent to obtain a suspension;
ii) filtering the suspension; and
iii) collecting and optionally concentrating the filtered suspension, to obtain a solution of the DO3A tri-tert-butyl ester **1A** in the organic solvent.

4. The process of claims 1-3 wherein step **2)** comprises reacting D-glucamine with epichlorohydrin, to obtain a solution of the compound of formula **2** in an organic solvent.

5. The process of claim 4 which comprises:
i) adding an aqueous solution of D-glucamine to a solution of epichlorohydrin in an organic solvent, to give the compound of formula **2** in a water/organic solvent mixture; preferably the amount of epichlorohydrin being in a slight excess over the stoichiometric amount; and
ii) removing the water from the mixture, to obtain a solution of the compound of formula **2** in the organic solvent.

6. The process of claims 4 or 5 wherein the organic solvent is Dimethylacetamide (DMAC).

7. The process of claims 1-6 wherein step **3)** comprises
a. reacting the compound of formula **2** from step **2)** with DO3A tri-tert-butyl ester **1A** from step **1)** in the presence of a base to give an organic crude solution;
b. diluting the obtained organic crude solution with water, a water/organic solvent mixture, or an aqueous solution to obtain a water/organic crude;
c. purifying the water/organic crude, to obtain the compound of formula **3** in a water/organic solvent mixture, and
d. optionally removing the organic solvent from the mixture to obtain an aqueous solution of the compound of formula **3.**

8. The process of claim 7 that comprises adding an aqueous solution to the water/organic crude of step b) resulting from dilution of the organic crude solution with a water/organic solvent mixture.

9. The process of claims 7-8 wherein the purification of the water/organic crude of step c) is performed by chromatography.

10. The process of any one of claims 1-9 wherein step **4)** comprises:
i) adding an acid to the aqueous solution of the compound of formula **3** from step **3)** to remove the protecting tert-butyl groups and obtain an acidic aqueous solution of the respective free ligand of formula **4;**
ii) adding a base to the acidic solution to obtain a substantially neutral aqueous solution of the free ligand;
iii) purifying and then optionally concentrating the obtained neutral solution to obtain an aqueous solution of the free ligand of formula **4.**

11. The process of claim 10 wherein step iii) comprises: distilling the neutral solution for removing the formed t-butanol; desalining the distillation residue; purifying by chromatography the desalinated solution to obtain an aqueous solution of the ligand of formula **4;** and optionally concentrating the aqueous solution.

12. The process of claims 10-11 wherein the obtained aqueous solution of the ligand of formula **4** has a concentration of 8-25% (w/w).

13. The process of any one of claims 1-12 wherein step **5)** comprises:
i) adding a gadolinium salt to the solution of the ligand of formula **4** from step **4)** to obtain a mixture comprising the dimeric complex of formula **5;**
ii) purifying the mixture, to obtain a solution of the dimeric complex; and
iii) concentrating the solution.

14. The process of claim 13 wherein step ii) comprises purifying the mixture by chromatography.

15. The process of claims 1-14 wherein step **6)** comprises isolating the dimeric complex of formula **5** as a white solid by spray drying of the solution directly collected from step **5).**

## Patentansprüche

1. Verfahren zur Herstellung der dimeren Komplexverbindung 5 der Formel 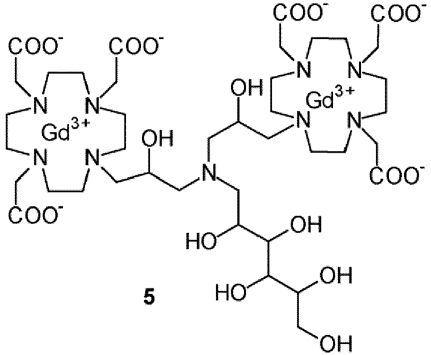 das Folgendes umfasst:
1) Herstellen einer Lösung von DO3A-Tri-tert-butylester der Formel **1A** 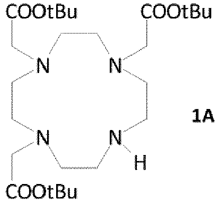 in einem organischen Lösungsmittel;
2) Herstellen einer Lösung einer Verbindung der Formel **2** 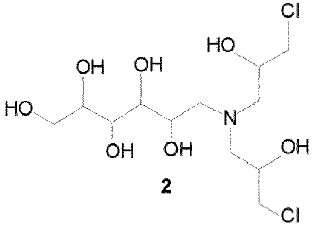 in einem organischen Lösungsmittel;
**3)** Mischen der gemäß den Schritten **1)** und **2)** hergestellten Lösungen zum Erhalt einer Lösung einer Verbindung der Formel **3** 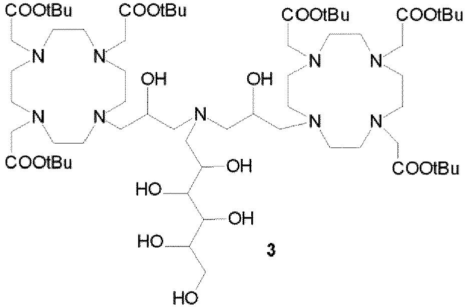
**4)** ohne Isolierung der Verbindung aus der Lösung von Schritt **3),** Entfernen der tert-Butyl-Schutzgruppen von der Verbindung der Formel **3** zum Erhalt einer Lösung eines jeweiligen freien Liganden der Formel **4** 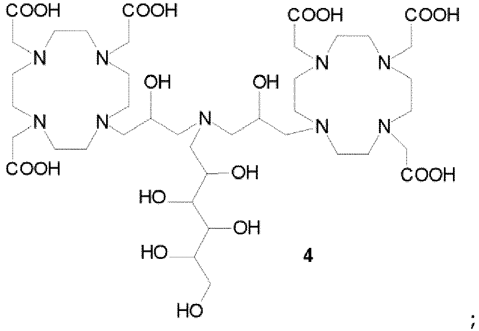
**5)** ohne Isolierung des freien Liganden der Formel **4,** Zugeben von Gadoliniummetallionen zu der Lösung von Schritt **4)** zum Erhalt einer Lösung des jeweiligen dimeren Komplexes der Formel 5; und
**6)** Isolieren des dimeren Komplexes,
wobei es sich bei dem Reaktionslösungsmittel in allen der Schritte nach der Herstellung der Verbindung der Formel **3** um ein wässriges Lösungsmittel handelt.

2. Verfahren nach Anspruch 1, wobei die Lösung des D03A-Tri-tert-butylesters der Formel **1A** von Schritt **1)** aus einem Hydrobromidsalz des DO3A-Tri-tert-butylesters hergestellt wird.

3. Verfahren nach Anspruch 1 oder 2, wobei Schritt **1)** Folgendes umfasst:
i) Suspendieren eines Hydrobromidsalzes des DO3A-Tri-tert-butylesters zusammen mit einer Base oder einem basischen Salz in einem organischen Lösungsmittel zum Erhalt einer Suspension;
ii) Filtrieren der Suspension; und
iii) Sammeln und gegebenenfalls Aufkonzentrieren der filtrierten Suspension zum Erhalt einer Lösung des D03A-Tri-tert-butylesters der Formel **1A** in dem organischen Lösungsmittel.

4. Verfahren nach den Ansprüchen 1-3, wobei Schritt **2)** das Umsetzen von D-Glucamin mit Epichlorhydrin zum Erhalt einer Lösung der Verbindung der Formel **2** in einem organischen Lösungsmittel umfasst.

5. Verfahren nach Anspruch 4, das Folgendes umfasst:
i) Zugeben einer wässrigen Lösung von D-Glucamin zu einer Lösung von Epichlorhydrin in einem organischen Lösungsmittel zum Erhalt der Verbindung der Formel **2** in einer Mischung von Wasser und organischem Lösungsmittel; wobei die Menge an Epichlorhydrin vorzugsweise in leichtem Überschuss gegenüber der stöchiometrischen Menge vorliegt; und
ii) Entfernen des Wassers aus der Mischung zum Erhalt einer Lösung der Verbindung der Formel 2 in dem organischen Lösungsmittel.

6. Verfahren nach Anspruch 4 oder 5, wobei es sich bei dem organischen Lösungsmittel um Dimethylacetamid (DMAC) handelt.

7. Verfahren nach den Ansprüchen 1-6, wobei Schritt **3)** Folgendes umfasst:
a. Umsetzen der Verbindung der Formel **2** aus Schritt **2)** mit D03A-Tri-tert-butylester der Formel **1A** aus Schritt **1)** in Gegenwart einer Base zum Erhalt einer organischen Rohproduktlösung;
b. Verdünnen der erhaltenen organischen rohen Lösung mit Wasser, einer Mischung von Wasser und organischem Lösungsmittel oder einer wässrigen Lösung zum Erhalt eines wässrig-organischen Rohprodukts;
c. Reinigen des wässrig-organischen Rohprodukts zum Erhalt der Verbindung der Formel **3** in einer Mischung von Wasser und organischem Lösungsmittel und
d. gegebenenfalls Entfernen des organischen Lösungsmittels aus der Mischung zum Erhalt einer wässrigen Lösung der Verbindung der Formel **3.**

8. Verfahren nach Anspruch 7, das das Zugeben einer wässrigen Lösung zu dem aus der Verdünnung der organischen Rohproduktlösung mit einer Mischung von Wasser und organischem Lösungsmittel resultierenden wässrig-organischen Rohprodukt von Schritt b) umfasst.

9. Verfahren nach den Ansprüchen 7-8, wobei die Reinigung des wässrig-organischen Rohprodukts von Schritt c) mittels Chromatographie durchgeführt wird.

10. Verfahren nach einem der Ansprüche 1-9, wobei Schritt **4)** Folgendes umfasst:
i) Zugeben einer Säure zu der wässrigen Lösung der Verbindung der Formel **3** aus Schritt **3)** zur Entfernung der tert-Butyl-Schutzgruppen und zum Erhalt einer sauren wässrigen Lösung des jeweiligen freien Liganden der Formel **4;**
ii) Zugeben einer Base zu der sauren Lösung zum Erhalt einer weitgehend neutralen wässrigen Lösung des freien Liganden;
iii) Reinigen und dann gegebenenfalls Aufkonzentrieren der erhaltenen neutralen Lösung zum Erhalt einer wässrigen Lösung des freien Liganden der Formel **4.**

11. Verfahren nach Anspruch 10, wobei Schritt iii) Folgendes umfasst: Destillieren der neutralen Lösung zur Entfernung des gebildeten t-Butanols; Entsalzen des Destillationsrückstands; Reinigen der entsalzten Lösung mittels Chromatographie zum Erhalt einer wässrigen Lösung des Liganden der Formel **4** und gegebenenfalls Aufkonzentrieren der wässrigen Lösung.

12. Verfahren nach den Ansprüchen 10-11, wobei die erhaltene wässrige Lösung des Liganden der Formel **4** eine Konzentration von 8-25 % (w/w) aufweist.

13. Verfahren nach einem der Ansprüche 1-12, wobei Schritt **5)** Folgendes umfasst:
i) Zugeben eines Gadoliniumsalzes zu der Lösung des Liganden der Formel **4** aus Schritt **4)** zum Erhalt einer Mischung, die den dimeren Komplex der Formel **5** umfasst;
ii) Reinigen der Mischung zum Erhalt einer Lösung des dimeren Komplexes; und
iii) Aufkonzentrieren der Lösung.

14. Verfahren nach Anspruch 13, wobei Schritt ii) das Reinigen der Mischung mittels Chromatographie umfasst.

15. Verfahren nach den Ansprüchen 1-14, wobei Schritt **6)** das Isolieren des dimeren Komplexes der Formel **5** in Form eines weißen Feststoffs durch Sprühtrocknen der direkt aus Schritt **5)** gesammelten Lösung umfasst.

## Revendications

1. Procédé de fabrication du composé de complexe dimère **5** de formule 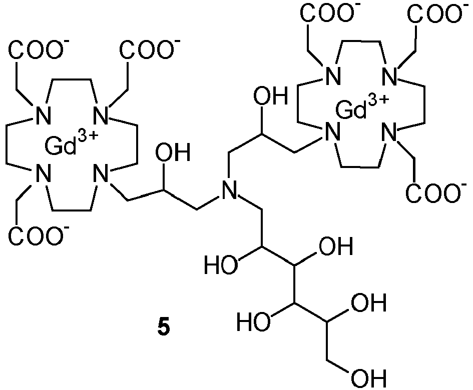 qui comprend :
**1)** la préparation d'une solution d'ester tri-tert-butylique de DO3A de formule **1A** 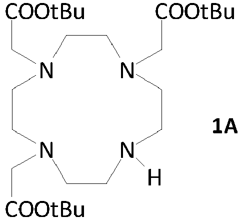 dans un solvant organique ;
**2)** la préparation d'une solution d'un composé de formule **2** 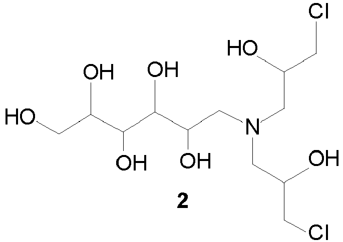 dans un solvant organique ;
**3)** le mélangeage des solutions préparées conformément aux étapes **1)** et **2)** pour obtenir une solution d'un composé de formule **3** 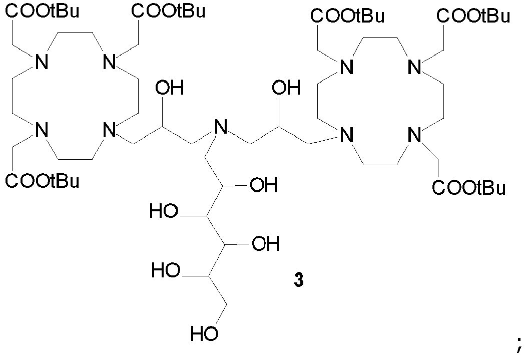
**4)** sans isolement du composé depuis la solution de l'étape **3),** le retrait des groupements protecteurs tert-butyle du composé de formule **3** pour obtenir une solution d'un ligand libre correspondant de formule **4** 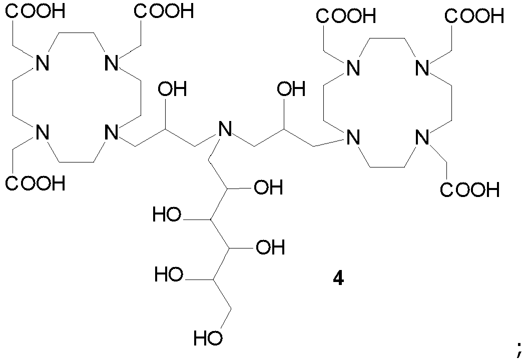
**5)** sans isolement du ligand libre de formule **4,** l'ajout d'ions métalliques de gadolinium à la solution de l'étape **4)** pour obtenir une solution du complexe dimère correspondant de formule **5** ; et
**6)** l'isolement du complexe dimère,
dans lequel le solvant réactionnel dans toutes les étapes suivant la préparation du composé de formule 3 est un solvant aqueux.

2. Procédé selon la revendication 1 dans lequel la solution de l'ester tri-tert-butylique de DO3A **1A** de l'étape **1)** est préparé à partir d'un sel de bromhydrate de l'ester tri-tert-butylique de DO3A.

3. Procédé selon les revendications 1 ou 2 dans lequel l'étape **1)** comprend :
i) la suspension d'un sel de bromhydrate de l'ester tri-tert-butylique de DO3A conjointement avec une base ou un sel basique dans un solvant organique pour obtenir une suspension ;
ii) la filtration de la suspension ; et
iii) le recueil et éventuellement la concentration de la suspension filtrée, pour obtenir une solution de l'ester tri-tert-butylique de DO3A **1A** dans le solvant organique.

4. Procédé selon les revendications 1 à 3 dans lequel l'étape **2)** comprend la réaction de la D-glucamine avec l'épichlorhydrine, pour obtenir une solution du composé de formule **2** dans un solvant organique.

5. Procédé selon la revendication 4 qui comprend :
i) l'ajout d'une solution aqueuse de D-glucamine à une solution d'épichlorhydrine dans un solvant organique, pour obtenir le composé de formule **2** dans un mélange eau/solvant organique ; préférentiellement la quantité d'épichlorhydrine étant en léger excès par rapport à la quantité stoechiométrique ; et
ii) le retrait de l'eau du mélange, pour obtenir une solution du composé de formule **2** dans le solvant organique.

6. Procédé selon les revendications 4 ou 5 dans lequel le solvant organique est le Diméthylacétamide (DMAC).

7. Procédé selon les revendications 1 à 6 dans lequel l'étape **3)** comprend
a. la réaction du composé de formule **2** issu de l'étape **2)** avec l'ester tri-tert-butylique de DO3A **1A** de l'étape **1)** en présence d'une base pour obtenir une solution organique brute ;
b. la dilution de la solution organique brute obtenue par de l'eau, un mélange eau/solvant organique ou une solution aqueuse pour obtenir un brut eau/organique ;
c. la purification du brut eau/organique, pour obtenir le composé de formule **3** dans un mélange eau/solvant organique, et
d. éventuellement le retrait du solvant organique du mélange pour obtenir une solution aqueuse du composé de formule **3.**

8. Procédé selon la revendication 7 qui comprend l'ajout d'une solution aqueuse au brut eau/organique de l'étape b) résultant de la dilution de la solution organique brute par un mélange eau/solvant organique.

9. Procédé selon les revendications 7 à 8 dans lequel la purification du brut eau/organique de l'étape c) est réalisée par chromatographie.

10. Procédé selon l'une quelconque des revendications 1 à 9 dans lequel l'étape **4)** comprend :
i) l'ajout d'un acide à la solution aqueuse du composé de formule **3** issu de l'étape **3)** pour retirer les groupements protecteurs tert-butyle et obtenir une solution aqueuse acide du ligand libre correspondant de formule **4** ;
ii) l'ajout d'une base à la solution acide pour obtenir une solution aqueuse essentiellement neutre du ligand libre ;
iii) la purification puis éventuellement la concentration de la solution neutre obtenue pour obtenir une solution aqueuse du ligand libre de formule **4.**

11. Procédé selon la revendication 10 dans lequel l'étape iii) comprend : la distillation de la solution neutre pour retirer le t-butanol formé ; la désalinisation du résidu de distillation ; la purification par chromatographie de la solution désalinisée pour obtenir une solution aqueuse du ligand de formule **4** ; et éventuellement la concentration de la solution aqueuse.

12. Procédé selon les revendications 10 à 11 dans lequel la solution aqueuse obtenue du ligand de formule **4** a une concentration de 8 à 25 % en masse.

13. Procédé selon l'une quelconque des revendications 1 à 12 dans lequel l'étape **5)** comprend :
i) l'ajout d'un sel de gadolinium à la solution du ligand de formule **4** issue de l'étape **4)** pour obtenir un mélange comprenant le complexe dimère de formule **5** ;
ii) la purification du mélange, pour obtenir une solution du complexe dimère ; et
iii) la concentration de la solution.

14. Procédé selon la revendication 13 dans lequel l'étape ii) comprend la purification du mélange par chromatographie.

15. Procédé selon les revendications 1 à 14 dans lequel l'étape **6)** comprend l'isolement du complexe dimère de formule **5** sous forme d'un solide blanc par séchage par atomisation de la solution directement recueillie dans l'étape **5).**
